# EUROPEAN PATENT SPECIFICATION

(11) **EP 4 489 588 B1**
(45) Date of publication and mention of the grant of the patent: **15.04.2026**
(21) Application number: 23711761.9
(22) Date of filing: 10.03.2023
(51) Int. Cl.: A23L 29/30, A23L 33/21, A61K 31/702, A61P 3/00, C07H 3/06, C12P 19/14, A23L 33/125, C12P 19/00

(54) **GALACTOOLIGOSACCHARIDE COMPOSITION**
GALAKTOOLIGOSACCHARID-ZUSAMMENSETZUNG
COMPOSITION DE GALACTO-OLIGOSACCHARIDE

(30) Priority: 11.03.2022 GB 202203449
(43) Date of publication of application: 15.01.2025
(73) Proprietor: Clasado Limited, Reading Berkshire RG2 0TD (GB)
(72) Inventor: BALTULIONIS, Gediminas, Reading Berkshire RG2 0TD (GB); LITHERLAND, Kelly, Reading Berkshire RG2 0TD (GB); HARTHOORN, Leunis Forrinus, Reading Berkshire RG2 0TD (GB); MARUSZAK, Aleksandra Elzbieta, Reading Berkshire RG2 0TD (GB); BASSON, Abigail, Reading Berkshire RG6 6AH (GB); WATSON, Kimberley, Reading Berkshire RG6 6AH (GB)
(74) Representative: Appleyard Lees IP LLP
(86) International application number: PCT/GB2023/050574
(87) International publication number: WO 2023/170426

(56) References cited:
- WO-A1-2019/119102
- WO-A1-2021/175878
- HUIFANG YIN ET AL: "Engineering of the Bacillus circulans &bgr;-Galactosidase Product Specificity", BIOCHEMISTRY, vol. 56, no. 5, 25 January 2017 (2017-01-25), pages 704 - 711, XP055416613, ISSN: 0006-2960, DOI: 10.1021/acs.biochem.7b00032
- YIN HUIFANG ET AL: "Synthesis of galacto-oligosaccharides derived from lactulose by wild-type and mutant [beta]-galactosidase enzymes from Bacillus circulans ATCC 31382", CARBOHYDRATE RESEARCH, vol. 465, 1 July 2018 (2018-07-01), GB, pages 58 - 65, XP093047838, ISSN: 0008-6215, DOI: 10.1016/j.carres.2018.06.009
- YIN HUIFANG ET AL: "Reaction kinetics and galactooligosaccharide product profiles of the [beta]-galactosidases fromBacillus circulans,Kluyveromyces lactisandAspergillus oryzae", FOOD CHEMISTRY, ELSEVIER LTD, NL, vol. 225, 7 January 2017 (2017-01-07), pages 230 - 238, XP029915071, ISSN: 0308-8146, DOI: 10.1016/J.FOODCHEM.2017.01.030

## Description

### Field

The present invention relates to a composition comprising oligosaccharide compounds, and use of said composition as a dietary supplement. In particular the present invention relates to an oligosaccharide composition with a relatively high amount of certain beneficial oligosaccharides.

### Background

Human gut microbiota (or microflora) comprises pathogenic, benign and beneficial microbial genera. A predominance of the former may lead to intestinal disorders that can be both acute (e.g. gastroenteritis) and chronic (e.g. inflammatory bowel disease, irritable bowel syndrome and some intestinal cancers). In humans, large intestinal microbiota is acquired at birth. Breast milk-fed infants have a preponderance of bifidobacteria, which easily outcompete other genera of bacteria. This is because human milk components stimulate growth of bifidobacteria. However, many factors can influence the composition and activity of intestinal microbiota over the course of an individual's lifetime, such as environment, drug treatments and diet, in particular the consumption of highly processed foods (see "Role of the gut microbiota in nutrition and health", Valdes, A. M. et al., BMJ, 2018, 361).

Certain components of the gut microbiota have been implicated in the aetiology of gut disease. For example, mycobacteria are associated with Crohn's disease; ulcerative colitis may be triggered by sulphate-reducing bacteria and there may be bacterial involvement in the development of bowel cancer. It would be of benefit if the selective growth of indigenous beneficial gut bacteria could be encouraged by the ingestion of a prebiotic. A prebiotic is a substrate that is selectively utilized by host microorganisms conferring a health benefit. Use of such a prebiotic may have the ongoing beneficial effect of supressing the growth of pathogenic microbiota.

Attempts have been made to influence the balance of the gut microbiota in favour of beneficial microorganisms, such as the bifidobacteria, by adding one or more such microbial strains to an appropriate food vehicle with the intention of conferring a health benefit on the host. Such a live microbial feed supplement is known as a probiotic. However, it is difficult to guarantee the survival of live bacteria in foods after digestion and consequently the actual effectiveness of probiotic may be limited. Therefore prebiotics may be a more promising option for positively affecting gut microbiota in a patient (see Walter J, Maldonado-Gómez MX, Martinez I. "To engraft or not to engraft: an ecological framework for gut microbiome modulation with live microbes", Curr Opin Biotechnol. 2018, 49, 129-139).

As noted above, an alternative approach to dietary manipulation of the gut microbiota is the use of a prebiotic, which is defined as a non-digestible food ingredient that beneficially affects the subject by selectively stimulating the growth and/or activity of one or a limited number of bacteria in the gut, thereby resulting in an improvement in the health of the host.

One group of compounds that are classified as prebiotics are the galactooligosaccharides, which are galactose-containing oligosaccharides produced from lactose by the transgalactosylase activity of β-galactosidase enzyme. Known galactooligosaccharide prebiotic products contain a mixture of many oligosaccharide compounds in varying proportions, not all of which have the desired beneficial effects on the intestinal microbiota of the consumer. Although such products have shown some beneficial results in improving the gastro-intestinal health of patients, there remains a need for further improved oligosaccharide compositions to fully realise the potential benefits to consumers of such prebiotics. Huifang Yin, Tjaard Pijning, Xiangfeng Meng, Lubbert Dijkhuizen & Sander S van Leeuwen, "Engineering of the Bacillus circulans β-Galactosidase Product Specificity", Biochemistry 2017, vol. 56, no. 5, pages 704-711, discloses a genetically engineered beta-galactosidase enzyme.

WO 2019/119102 A1 discloses the preparation of a GOS syrup. Yin Huifang, Lubbert Dijkhuizen & Sander S. van Leeuwen "Synthesis of galacto-oligosaccharides derived from lactulose by wild-type and mutant β-galactosidase enzymes from Bacillus circulans ATCC 31382", Carbohydrate Research 2018, vol. 465, pages 58-65, discloses a mutant galactosidase. Yin Huifang, Jelle B. Bultema, Lubbert Dijkhuizen, Sander S. van Leeuwen "Reaction kinetics and galactooligosaccharide product profiles of the β-galactosidases from Bacillus circulans, Kluyveromyces lactis and Aspergillus oryzae" Food Chemistry, Elsevier LTD, NL, 2017, vol. 225, pages 230-238, discloses a study of different GOS profiles prepared with different commercial enzymes. WO 2021/175878 discloses the preparation of GOS with mannose wherein lactose is first converted into epilactose using an epimerase enzyme. The composition and use of said composition according to the present invention is not disclosed nor suggested in any of the above documents.

### Summary of the Invention

The invention is set out in the appended claims. It is one aim of the present invention, amongst others, to provide a composition comprising oligosaccharide compounds that addresses at least one disadvantage of the prior art, whether identified here or elsewhere, or to provide an alternative to existing compositions. For instance it may be an aim of the present invention to provide a composition comprising oligosaccharide compounds which comprise a higher proportion of specific beneficial oligosaccharides than known oligosaccharide compositions.

According to aspects of the present invention, there is provided a composition and use as set forth in the appended claims. Other features of the invention will be apparent from the dependent claims, and from the description which follows.

According to a first aspect of the present invention, there is provided a composition comprising oligosaccharide compounds, wherein the oligosaccharide compounds comprise:
(a) at least 8 wt% Gal-(β1-3)-Gal-(β1-4)-X^{a};
(b) at least 3 wt% Gal-(β1-3)-Gal-(β1-3)-X^{b}; and
(c) at least 5 wt% Gal-(β1-3)-Gal-(β1-2)-X^{c},

based on the total weight of oligosaccharide compounds present in the composition;
wherein X^{a}, X^{b} and X^{c} are each independently selected from monosaccharides.

These types of oligosaccharides having the specified linkages between the galactose units (denoted Gal) and terminal monosaccharides are believed to be particularly beneficial for the maintenance of a healthy human gut microbiota and therefore beneficial to the health of a subject. Known oligosaccharide compositions either do not contain each of these types of oligosaccharides or contain lower amounts of these oligosaccharides. Therefore the compositions of this first aspect are believed to provide advantages to the consumer in the establishment and maintenance of human gut microbiota when used as prebiotics.

The weight percentages of the specific oligosaccharide compounds discussed herein are based on the weight of all of the oligosaccharide compounds present in the whole composition. Therefore only the fraction of the composition which is provided by oligosaccharides, either components (a), (b), (c) or any other oligosaccharide present, is taken into account when determining the specified weight percentages. For this determination of oligosaccharide content, disaccharides are included, apart from lactose. The composition of this first aspect may contain other non-oligosaccharide components, including monosaccharides and lactose. These components are not taken into account when determining the specified weight percentages of the oligosaccharide compounds discussed herein. The specified amounts of oligosaccharide compounds in the composition can be referred to as a weight percentage of the oligosaccharide fraction of the composition of this first aspect.

Oligosaccharide compounds (a), (b) and (c) comprise X^{a}, X^{b} and X^{c} respectively, which are each independently selected from saccharides. The X^{a}, X^{b} and X^{c} groups may be considered to be saccharide units. Therefore the groups X^{a}, X^{b} and X^{c} can be considered terminal sugars of the oligosaccharide compounds. The groups X^{a}, X^{b} and X^{c} are suitably independently selected from monosaccharides. Any suitable monosaccharide unit which can form oligosaccharides with the galactose units of compounds (a), (b) and (c) can provide X^{a}, X^{b} and X^{c}. Suitably X^{a}, X^{b} and X^{c} are each independently selected from the following monosaccharides. Suitable monosaccharide units are selected from glucose (Glc), fucose (Fuc), arabinose (Ara), xylose (Xyl), rhamnose (Rha), mannose (Man), galactose (Gal), ribose (Rib), lyxose (Lyx), allose (All), altrose (Alt), gulose (Gul), idose (Ido), talose (Tal), psicose (Psi), fructose (Fru), sorbose (Sor), tagatose (Tag), galactosamine (GalN), glucosamine (GlcN) and N-Acetylglucosamine (GlcNAc) or a mixture thereof. Therefore each of compounds (a), (b) and (c) may comprise a mixture of oligosaccharide compounds having different respective X groups, for example either Glc or Fuc X groups.

In some embodiments, groups X^{a}, X^{b} and X^{c} are independently selected from the monosaccharides listed above, suitably independently selected from Glc, Fuc, Ara, Xyl, Rha and Man or a mixture thereof.

The saccharide units of the oligosaccharides in the composition of this first aspect may have either the D or the L enantiomeric form. Suitably the Gal saccharide units in components (a), (b) and (c) all have the D enantiomeric form. The components (a), (b) and (c) may therefore be as follows:
(a) D-Gal-(β1-3)-D-Gal-(β1-4)-X^{a};
(b) D-Gal-(β1-3)-D-Gal-(β1-3)-X^{b}; and
(c) D-Gal-(β1-3)-D-Gal-(β1-2)-X^{c}.

Suitably the Gal and the X^{a}, X^{b} and X^{c} saccharide units all have the D enantiomeric form. The components (a), (b) and (c) may therefore be as follows:
(a) D-Gal-(β1-3)-D-Gal-(β1-4)-D-X^{a};
(b) D-Gal-(β1-3)-D-Gal-(β1-3)-D-X^{b}; and
(c) D-Gal-(β1-3)-D-Gal-(β1-2)-D-X^{c}.

In some embodiments, each of X^{a}, X^{b} and X^{c} are Glc. Therefore compounds (a), (b) and (c) may be galactooligosaccharide compounds (GOS) and the composition of this first aspect may be referred to as a galactooligosaccharide composition. Such galactooligosaccharides may be formed by converting lactose to the stated oligosaccharides with a suitable galactosidase enzyme. In such embodiments the components (a), (b) and (c) are suitably as follows:
(a) Gal-(β1-3)-Gal-(β1-4)-Glc;
(b) Gal-(β1-3)-Gal-(β1-3)-Glc; and
(c) Gal-(β1-3)-Gal-(β1-2)-Glc.

Suitably the Gal and the Glc saccharide units all have the D enantiomeric form. The components (a), (b) and (c) may therefore be as follows:
(a) D-Gal-(β1-3)-D-Gal-(β1-4)-D-Glc;
(b) D-Gal-(β1-3)-D-Gal-(β1-3)-D-Glc; and
(c) D-Gal-(β1-3)-D-Gal-(β1-2)-D-Glc.

In some embodiments, each of X^{a}, X^{b} and X^{c} are a mixture of Glc and one or more of other saccharide units described above, for example Fuc, Ara, Xyl, Rha and Man. Therefore each of (a), (b) and (c) may comprise a mixture of oligosaccharide compounds having either Glc or one of the other saccharide units described above as the X group. In such embodiments, components (a), (b) and (c) may be formed by converting a mixture of lactose and an appropriate additional sugar, for example a monosaccharide selected from fucose, arabinose, xylose, rhamnose and mannose, to the oligosaccharides. Suitably each of X^{a}, X^{b} and X^{c} are a mixture of Glc and Fuc.

Other oligosaccharides in the composition comprising oligosaccharide compounds of this first aspect, besides components (a), (b) and (c) discussed above, may also comprise the saccharide unit "X" groups referred to above.

The composition of this first aspect comprises (a) at least 8 wt% Gal-(β1-3)-Gal-(β1-4)-X^{a}. Suitably the composition comprises at least 9 wt% of component (a) or at least 10 wt% of component (a).

Suitably the composition comprises up to 35 wt% of component (a), up to 30 wt% of component (a) or up to 25 wt% of component (a).

Suitably the composition comprises from 8 to 35 wt% of component (a), from 8 to 25 wt% of component (a) or from 10 to 20 wt% of component (a).

The composition of this first aspect comprises (b) at least 3 wt% Gal-(β1-3)-Gal-(β1-3)-X^{b}. Suitably the composition comprises at least 4 wt% of component (b) or at least 5 wt% of component (b). Suitably the composition comprises at least 6 wt% of component (b) or at least 8 wt% of component (b).

Suitably the composition comprises up to 25 wt% of component (b), up to 20 wt% of component (b) or up to 15 wt% of component (b).

Suitably the composition comprises from 3 to 25 wt% of component (b), from 4 to 20 wt% of component (b) or from 4 to 10 wt% of component (b).

The composition of this first aspect comprises (c) at least 5 wt% Gal-(β1-3)-Gal-(β1-2)-X^{c}. Suitably the composition comprises at least 6 wt% of component (c). Suitably the composition comprises at least 8 wt% of component (c).

Suitably the composition comprises up to 25 wt% of component (c), up to 20 wt% of component (c) or up to 15 wt% of component (c).

Suitably the composition comprises from 5 to 25 wt% of component (c), from 5 to 20 wt% of component (c) or from 6 to 10 wt% of component (c).

The above amounts are based on the total weight of oligosaccharide compounds present in the composition.

Suitably in the composition of this first aspect:
component (a) is present in an amount of at least 10 wt%;
component (b) is present in an amount of at least 6 wt%; and
component (c) is present in an amount of at least 6 wt%;
based on the total weight of oligosaccharide compounds present in the composition.

Suitably in the composition of this first aspect:
component (a) is present in an amount up to 35 wt%;
component (b) is present in an amount up to 25 wt%; and
component (c) is present in an amount up to 25 wt%;
based on the total weight of oligosaccharide compounds present in the composition.

Suitably in the composition of this first aspect:
component (a) is present in an amount from 8 to 25 wt%;
component (b) is present in an amount from 3 to 25 wt%; and
component (c) is present in an amount from 5 to 20 wt%;
based on the total weight of oligosaccharide compounds present in the composition.

Suitably in the composition of this first aspect:
component (a) is present in an amount from 8 to 25 wt%;
component (b) is present in an amount from 6 to 25 wt%; and
component (c) is present in an amount from 6 to 20 wt%;
based on the total weight of oligosaccharide compounds present in the composition.

In some embodiments, the composition of this first aspect comprises:
(a) from 8 to 25 wt% Gal-(β1-3)-Gal-(β1-4)-Glc;
(b) from 3 to 25 wt% Gal-(β1-3)-Gal-(β1-3)-Glc; and
(c) from 5 to 20 wt% Gal-(β1-3)-Gal-(β1-2)-Glc,
based on the total weight of oligosaccharide compounds present in the composition.

In some embodiments, the composition of this first aspect comprises:
(a) from 8 to 25 wt% Gal-(β1-3)-Gal-(β1-4)-Glc;
(b) from 6 to 25 wt% Gal-(β1-3)-Gal-(β1-3)-Glc; and
(c) from 6 to 20 wt% Gal-(β1-3)-Gal-(β1-2)-Glc,
based on the total weight of oligosaccharide compounds present in the composition.

Suitably the ratio of the wt% of compound (a) to compound (b) is from 1:1 to 3:1, suitably from 1.5:1 to 2.5:1.

Suitably the ratio of the wt% of compound (a) to compound (c) is from 1:1 to 3:1, suitably from 1.5:1 to 2.5:1.

Suitably the ratio of the wt% of compound (b) to compound (c) is from 2:1 to 1:2, suitably from 1.5:1 to 1:1.5.

Suitably, in the composition of this first aspect, the oligosaccharide compounds comprise:
(d) at least 3 wt% Gal-(β1-3)-Gal-(β1-3)-Gal-(β1-4)-X^{d},
based on the total weight of oligosaccharide compounds present in the composition;
wherein X^{d}, is selected from saccharides.
X^{d} may be selected from the same monosaccharide units described above for X^{a}, X^{b} and X^{c}.

In some embodiments, X^{d} is Glc.

Suitably the Gal saccharide units of component (d) have the D enantiomeric form. Suitably the Gal and the X^{d} saccharide units all have the D enantiomeric form. The components (d) may therefore be as follows: D-Gal-(β1-3)-D-Gal-(β1-3)-D-Gal-(β1-4)-D-X^{d}.

In some embodiments, X^{d} is a mixture of Glc and one or more of other monosaccharide units, for example Fuc, Ara, Xyl, Rha and Man, suitably Fuc.

Suitably the composition comprises at least 4 wt% of component (d) or at least 5 wt% of component (d).

Suitably the composition comprises up to 25 wt% of component (d), up to 20 wt% of component (d) or up to 15 wt% of component (d).

Suitably the composition comprises from 3 to 25 wt% of component (d), from 4 to 20 wt% of component (d) or from 4 to 10 wt% of component (d).

Suitably the amount of component (d) present in the composition is lower than the amount of component (c) present in the composition.

Suitably in the composition of this first aspect:
component (a) is present in an amount from 8 to 25 wt%;
component (b) is present in an amount from 3 to 25 wt%;
component (c) is present in an amount from 5 to 20 wt%; and
component (d) is present in an amount from 3 to 25 wt%;
based on the total weight of oligosaccharide compounds present in the composition.

In some embodiments, the composition of this first aspect comprises:
(a) from 8 to 25 wt% Gal-(β1-3)-Gal-(β1-4)-Glc;
(b) from 3 to 25 wt% Gal-(β1-3)-Gal-(β1-3)-Glc;
(c) from 5 to 20 wt% Gal-(β1-3)-Gal-(β1-2)-Glc, and
(d) from 3 to 25 wt% Gal-(β1-3)-Gal-(β1-3)-Gal-(β1-4)-Glc,
based on the total weight of oligosaccharide compounds present in the composition.

Suitably, in the composition of this first aspect, the oligosaccharide compounds comprise:
(e) at least 5 wt% Gal-(β1-4)-Gal-(β1-4)-X^{e};
based on the total weight of oligosaccharide compounds present in the composition;
wherein X^{e} is selected from monosaccharides.
X^{e} may be selected from the same monosaccharide units described above for X^{a}, X^{b} and X^{c}.

Suitably the Gal saccharide units of component (e) have the D enantiomeric form. Suitably the Gal and the X^{e} saccharide units all have the D enantiomeric form. The components (e) may therefore be as follows: D-Gal-(β1-4)-D-Gal-(β1-4)-D-X^{e}.

In some embodiments, X^{e} is Glc.

In some embodiments, X^{e} is a mixture of Glc and one or more of other monosaccharide units, for example Fuc, Ara, Xyl, Rha and Man, suitably Fuc.

Suitably the composition comprises at least 6 wt% of component (e) or at least 7 wt% of component (e).

Suitably the composition comprises up to 25 wt% of component (e), up to 20 wt% of component (e) or up to 15 wt% of component (e).

Suitably the composition comprises from 5 to 25 wt% of component (e), from 5 to 20 wt% of component (e) or from 6 to 10 wt% of component (e).

Suitably the amount of component (e) present in the composition is lower than the amount of component (a) present in the composition.

Suitably in the composition of this first aspect:
component (a) is present in an amount from 8 to 25 wt%;
component (b) is present in an amount from 3 to 25 wt%;
component (c) is present in an amount from 5 to 20 wt%;
component (d) is present in an amount from 3 to 25 wt%; and
component (e) is present in an amount from 5 to 25 wt%;
based on the total weight of oligosaccharide compounds present in the composition.

In some embodiments, the composition of this first aspect comprises:
(a) from 8 to 25 wt% Gal-(β1-3)-Gal-(β1-4)-Glc;
(b) from 3 to 25 wt% Gal-(β1-3)-Gal-(β1-3)-Glc;
(c) from 5 to 20 wt% Gal-(β1-3)-Gal-(β1-2)-Glc, and
(d) from 3 to 25 wt% Gal-(β1-3)-Gal-(β1-3)-Gal-(β1-4)-Glc, and
(e) from 5 to 25 wt% Gal-(β1-4)-Gal-(β1-4)-Glc;
based on the total weight of oligosaccharide compounds present in the composition.

In the compositions of this first aspect, X^{a}, X^{b}, X^{c}, X^{d} and X^{e} are each independently selected from the monosaccharides described above.

In the compositions of this first aspect, X^{a}, X^{b}, X^{c}, X^{d} and X^{e} may each be independently selected from Glc, Fuc, Ara, Xyl, Rha and Man or a mixture thereof.

In some embodiments, X^{a}, X^{b}, X^{c}, X^{d} and X^{e} are each Glc.

In some embodiments, X^{a}, X^{b}, X^{c}, X^{d} and X^{e} each comprise Fuc. Suitably X^{a}, X^{b}, X^{c}, X^{d} and X^{e} are each a mixture of Glc and Fuc.

The oligosaccharide compounds of the compositions of this first aspect suitably have a relatively high proportion of β1-3 Gal-Gal linkages - mainly due to the presence of the components (a), (b), (c) and optionally (d) in the proportions discussed herein. The inventors have found that such relatively high proportions of β1-3 Gal-Gal linkages may be particularly advantageous for the uses of the composition discussed herein.

Suitably from 35 to 55% of the Gal-Gal linkages in the oligosaccharide compounds are 1-3 linkages, suitably from 40 to 55%, suitably from 40 to 50%.

Suitably from 40 to 60% of the Gal-Gal linkages in the oligosaccharide compounds are 1-4 linkages, suitably from 45 to 55%, suitably from 45 to 52%.

Suitably from 8 to 20% of the Gal-X linkages in the oligosaccharide compounds are 1-3 linkages, suitably from 10 to 18%, suitably from 12 to 16%, suitably wherein X is Glc.

Suitably from 15 to 25% of the Gal-X linkages in the oligosaccharide compounds are 1-4 linkages, suitably from 16 to 24%, suitably from 17 to 22%, suitably wherein X is Glc.

Suitably from 30 to 45% of the Gal-X linkages in the oligosaccharide compounds are 1-2 linkages, suitably from 32 to 43%, suitably from 34 to 41%, suitably wherein X is Glc.

Suitably from 20 to 36% of the Gal-X linkages in the oligosaccharide compounds are 1-6 linkages, suitably from 22 to 34%, suitably from 25 to 32%, suitably wherein X is Glc.

Suitably the Gal-X, linkages referred to above, for example for example Gal-Glu linkages, are β linkages, i.e β-glycosidic bonds.

Suitably the composition comprises at least 25 wt% of trisaccharides, based on the total weight of in the composition. Suitably the composition comprises at least 28 wt% of trisaccharides or at least 30 wt% of trisaccharides.

Suitably the composition comprises up to 70 wt% of trisaccharides, suitably up to 60 wt% trisaccharides or up to 50 wt% trisaccharides.

Suitably the composition comprises from 25 to 70 wt% of trisaccharides, suitably from 30 to 60 wt% trisaccharides or from 30 to 50 wt% trisaccharides.

Suitably the composition comprises at least 10 wt% of tetrasaccharides, based on the total weight of in the composition. Suitably the composition comprises at least 12 wt% of tetrasaccharides or at least 15 wt% of tetrasaccharides. Suitably the composition comprises at least 20 wt% tetrasaccharides or at least 25 wt% tetrasaccharides.

Suitably the composition comprises up to 40 wt% of tetrasaccharides, suitably up to 25 wt% tetrasaccharides or up to 20 wt% tetrasaccharides.

Suitably the composition comprises from 10 to 40 wt% of tetrasaccharides, suitably from 10 to 30 wt% tetrasaccharides or from 12 to 25 wt% tetrasaccharides.

Suitably the composition comprises from 30 to 50 wt% trisaccharides and from 10 to 25 wt% tetrasaccharides, based on the total weight of the composition.

As mentioned above, the determination of disaccharides present in the composition excludes lactose Therefore in some embodiments the composition suitably comprises up to 40 wt% of disaccharides, suitably up to 30 wt% disaccharides or up to 20 wt% disaccharides. Suitably the content of lactose in the composition of this first aspect is minimised. Suitably the composition is substantially free of lactose. Suitably the composition does not contain lactose.

Suitably the composition comprises from 10 to 40 wt% of disaccharides, suitably from 10 to 30 wt% disaccharides or from 10 to 20 wt% disaccharides.

Suitably the composition comprises from 10 to 40 wt% disaccharides, from 30 to 60 wt% trisaccharides and from 10 to 25 wt% tetrasaccharides, based on the total weight of the composition.

The following description relates to the content of disaccharides in the oligosaccharide compounds. Suitably the oligosaccharide compounds comprise up to 40 wt% of disaccharides, suitably up to 30 wt% disaccharides or up to 20 wt% disaccharides.

Suitably the oligosaccharide compounds comprise from 0 to 40 wt% of disaccharides, suitably from 10 to 30 wt% disaccharides or from 10 to 20 wt% disaccharides.

Suitably the oligosaccharide compounds comprise at least 25 wt% of trisaccharides, based on the total weight of oligosaccharide compounds present in the composition. Suitably the oligosaccharide compounds comprise at least 30 wt% of trisaccharides or at least 33 wt% of trisaccharides.

Suitably the oligosaccharide compounds comprise up to 75 wt% of trisaccharides, suitably up to 65 wt% trisaccharides or up to 55 wt% trisaccharides.

Suitably the oligosaccharide compounds comprise from 25 to 75 wt% of trisaccharides, suitably from 30 to 65 wt% trisaccharides or from 34 to 55 wt% trisaccharides.

Suitably the oligosaccharide compounds comprise at least 10 wt% of tetrasaccharides, based on the total weight of oligosaccharide compounds present in the composition. Suitably the oligosaccharide compounds comprise at least 12 wt% of tetrasaccharides or at least 15 wt% of tetrasaccharides.

Suitably the oligosaccharide compounds comprise up to 45 wt% of tetrasaccharides, suitably up to 35 wt% tetrasaccharides or up to 30 wt% tetrasaccharides.

Suitably the oligosaccharide compounds comprise from 10 to 45 wt% of tetrasaccharides, suitably from 10 to 35 wt% tetrasaccharides or from 15 to 30 wt% tetrasaccharides.

Suitably the oligosaccharide compounds comprise from 0 to 40 wt% disaccharides and from 30 to 75 wt% trisaccharides, based on the total weight of oligosaccharide compounds present in the composition.

Suitably the oligosaccharide compounds comprise from 0 to 40 wt% disaccharides, from 30 to 75 wt% trisaccharides and from 10 to 45 wt% tetrasaccharides, based on the total weight of oligosaccharide compounds present in the composition.

The composition of this first aspect suitably comprises at least 50 wt% of oligosaccharide compounds, including components (a), (b), (c) and optionally (d) and (e). Suitably the composition comprises at least 55 wt% of oligosaccharide compounds, suitably at least 60 wt%, based on the total weight of the composition.

Suitably the composition comprises up to 100 wt% oligosaccharide compounds, suitably up to 95 wt%, up to 90 wt% or up to 85 wt% oligosaccharide compounds.

Suitably the composition comprises from 50 to 100 wt% oligosaccharide compounds, suitably from 55 to 95 wt%, or from 60 to 85 wt% oligosaccharide compounds.

In some embodiments, the composition is in the form of a syrup. The syrup suitably comprises at least 50 wt% of oligosaccharide compounds, at least 55 wt% of oligosaccharide compounds, or at least 60 wt%. Suitably the syrup comprises from 50 to 75 wt% of oligosaccharide compounds, suitably from 55 to 70 wt% or from 60 to 70 wt% oligosaccharide compounds.

The syrup may comprise a significant amount of monosaccharides, for example glucose and/or galactose. The syrup may comprise from 15 to 30 wt% monosaccharides, suitably from 20 to 28 wt% monosaccharides or from 21 to 25 wt% monosaccharides, for example glucose and/or galactose.

The syrup suitably comprises from 20 to 30 wt% water, suitably from 22 to 28 wt% water.

The syrup may also contain lactose, for example 4 to 14 wt% lactose.

In some embodiments, the composition is in the form of a powder. The powder suitably comprises at least 60 wt% of oligosaccharide compounds, suitably at least 70 wt% or at least 75 wt% of oligosaccharide compounds. Suitably the powder comprises from 60 to 100 wt% of oligosaccharide compounds, suitably from 70 to 95 wt% or from 75 to 90 wt% oligosaccharide compounds.

The powder suitably comprises a reduced amount of monosaccharides, for example glucose and/or galactose, compared to the syrup discussed above. The powder may comprise from 1 to 10 wt% monosaccharides, suitably from 2 to 8 wt% monosaccharides or from 3 to 7 wt% monosaccharides, suitably approximately 5 wt%, for example of glucose and/or galactose.

The powder suitably comprises from 1 to 10 wt% water, suitably from 3 to 6 wt% water.

The composition of this first aspect may have been purified to remove monosaccharides and optionally disaccharides from the composition.

The composition of this first aspect may have been fractionated to separate the oligosaccharide components of the composition according to their molecular weight, for example to remove disaccharides from the composition or to isolate trisaccharides from the other oligosaccharide components. This may be carried out by any suitable method known in the art, for example high-performance liquid chromatography. The composition produced by such a fractionation may be referred to as an oligosaccharide fraction or a GOS fraction.

In such embodiments, the composition (or oligosaccharide fraction) suitably comprises at least 70 wt% of trisaccharides, tetrasaccharides and higher oligosaccharides, suitably at least 80 wt% or at least 90 wt%. Such higher oligosaccharides have a degree of polymerisation of 5 and above. Suitably the composition comprises at least 95 wt% of trisaccharides, tetrasaccharides and higher oligosaccharides. Suitably the composition consists or consists essentially of trisaccharides, tetrasaccharides and higher oligosaccharides.

In such embodiments, the composition suitably comprises from 40 to 70 wt% of trisaccharides, suitably from 45 to 70 wt% trisaccharides or from 50 to 70 wt% trisaccharides.

Suitably the oligosaccharide compounds comprise from 15 to 50 wt% of tetrasaccharides, suitably from 15 to 40 wt% tetrasaccharides or from 20 to 40 wt% tetrasaccharides.

Suitably the oligosaccharide compounds comprise from 5 to 25 wt% of higher oligosaccharides, suitably from 5 to 20 wt% higher oligosaccharides or from 10 to 20 wt% higher oligosaccharides.

Suitably the composition comprises from 40 to 70 wt% trisaccharides, from 15 to 40 wt% tetrasaccharides and 5 to 25 wt% of higher oligosaccharides, based on the total weight of the composition. Suitably the composition comprises from 50 to 70 wt% trisaccharides, from 20 to 40 wt% tetrasaccharides and from 10 to 20 wt% higher oligosaccharides, based on the total weight of the composition.

In such embodiments the composition (or oligosaccharide fraction) suitably comprises the components (a), (b) and (c) as described above in the following amounts:
(a) at least 8 wt% Gal-(β1-3)-Gal-(β1-4)-X^{a};
(b) at least 3 wt% Gal-(β1-3)-Gal-(β1-3)-X^{b}; and
(c) at least 5 wt% Gal-(β1-3)-Gal-(β1-2)-X^{c},

based on the total weight of oligosaccharide compounds present in the composition;
wherein X^{a}, X^{b} and X^{c} are each independently selected from monosaccharides.

Suitably the composition (or oligosaccharide fraction) comprises the components (a), (b) and (c) as described above in the following amounts:
(a) at least 10 wt% Gal-(β1-3)-Gal-(β1-4)-X^{a};
(b) at least 5 wt% Gal-(β1-3)-Gal-(β1-3)-X^{b}; and
(c) at least 7 wt% Gal-(β1-3)-Gal-(β1-2)-X^{c}.

The composition may comprise components (d) and/or component (e) as described above.

The composition suitably comprises said components in the ratios discussed above.

In some embodiments, the composition of this first aspect is a trisaccharide and tetrasaccharide fractionated product (which may be referred to as a DP3/DP4 fraction).

In such embodiments, the composition (or DP3/DP4 fraction) suitably comprises at least 70 wt% of trisaccharides and tetrasaccharides, suitably at least 80 wt% or at least 90 wt%. Suitably the composition consists or consists essentially of trisaccharides and tetrasaccharides.

In such embodiments, the composition suitably comprises from 50 to 80 wt% of trisaccharides, suitably from 55 to 75 wt% trisaccharides or from 60 to 75 wt% trisaccharides.

Suitably the oligosaccharide compounds comprise from 20 to 50 wt% of tetrasaccharides, suitably from 25 to 45 wt% tetrasaccharides or from 25 to 40 wt% tetrasaccharides.

Suitably the composition comprises from 50 to 80 wt% trisaccharides and from 20 to 50 wt% tetrasaccharides, based on the total weight of the composition. Suitably the composition comprises from 60 to 75 wt% trisaccharides and from 25 to 40 wt% tetrasaccharides, based on the total weight of the composition.

In such embodiments the composition (or oligosaccharide fraction) suitably comprises the components (a), (b) and (c) as described above in the following amounts:
(a) at least 8 wt% Gal-(β1-3)-Gal-(β1-4)-X^{a};
(b) at least 3 wt% Gal-(β1-3)-Gal-(β1-3)-X^{b}; and
(c) at least 5 wt% Gal-(β1-3)-Gal-(β1-2)-X^{c},

based on the total weight of oligosaccharide compounds present in the composition;
wherein X^{a}, X^{b} and X^{c} are each independently selected from monosaccharides.

Suitably the composition (or oligosaccharide fraction) comprises the components (a), (b) and (c) as described above in the following amounts:
(a) at least 10 wt% Gal-(β1-3)-Gal-(β1-4)-X^{a};
(b) at least 5 wt% Gal-(β1-3)-Gal-(β1-3)-X^{b}; and
(c) at least 7 wt% Gal-(β1-3)-Gal-(β1-2)-X^{c}.

The composition may comprise components (d) and/or component (e) as described above.

The composition suitably comprises said components in the ratios discussed above.

In some embodiments, the composition of this first aspect is a trisaccharide fractionated product (which may be referred to as a DP3 fraction). Such a trisaccharide fractionated product may be obtained by the known fractionation methods referred to above. Such a composition suitably comprises at least 70 wt% of trisaccharides, suitably at least 80 wt% or at least 90 wt%. Suitably the composition comprises at least 95 wt% of trisaccharides.

In such embodiments the composition (or oligosaccharide fraction) suitably comprises the components (a), (b) and (c) as described above in the following amounts:
(a) at least 8 wt% Gal-(β1-3)-Gal-(β1-4)-X^{a};
(b) at least 3 wt% Gal-(β1-3)-Gal-(β1-3)-X^{b}; and
(c) at least 5 wt% Gal-(β1-3)-Gal-(β1-2)-X^{c},

based on the total weight of oligosaccharide compounds present in the composition;
wherein X^{a}, X^{b} and X^{c} are each independently selected from saccharides.

Suitably the composition (or oligosaccharide fraction) comprises the components (a), (b) and (c) as described above in the following amounts:
(a) at least 10 wt% Gal-(β1-3)-Gal-(β1-4)-X^{a};
(b) at least 5 wt% Gal-(β1-3)-Gal-(β1-3)-X^{b}; and
(c) at least 7 wt% Gal-(β1-3)-Gal-(β1-2)-X^{c}.

The composition may comprise component (e) as described above.

The composition suitably comprises said components in the ratios discussed above.

The composition of this first aspect may be incorporated into a product for ingestion by a consumer, for improving gut health by promoting the growth of beneficial bacteria, such as bifidobacteria in the gut of the consumer. Such a product may be selected from the group consisting of dairy products (for example, liquid milk, dried milk powder such as whole milk powder, skimmed milk powder, fat filled milk powders, whey powders, infant formula, ice cream, yoghurt, cheese, fermented dairy products), beverages, infant foods, cereals, bread, biscuits, confectionary, cakes, food supplements, dietary supplements, animal feeds, poultry feeds or indeed any other food or beverage.

The composition of this first aspect may be incorporated into a synbiotic composition. Such a synbiotic composition is suitably a mixture comprising live microorganisms and substrate(s) selectively utilized by host microorganisms that confers a health benefit on the host, i.e. a probiotic and a prebiotic.

According to a second aspect of the present invention, there is provided a pharmaceutical or nutraceutical composition comprising a composition according to the first aspect and at least one carrier, excipient, or diluent.

The composition of the first aspect comprised in the pharmaceutical or nutraceutical composition is a composition comprising oligosaccharide compounds as described above. The composition comprising oligosaccharide compounds may have any of the suitable features and advantages described above in relation to the first aspect.

Suitable further components of pharmaceutical or nutraceutical compositions and methods of preparing such pharmaceutical or nutraceutical compositions are known in the art.

According to a third aspect of the present invention, there is provided a use of the composition according to the first aspect as a dietary supplement.

The composition of the first aspect is a composition comprising oligosaccharide compounds, as described above. The composition comprising oligosaccharide compounds may have any of the suitably features and advantages described above in relation to the first aspect.

The use of this third aspect suitably increases butyrate production in the gut of a consumer of the dietary supplement. The term butyrate is used to refer to butyric acid and its derivatives, such as salts and esters of butyric acid which are produced by gut microbiota in mammals. Specifically, members of the *Bifidobacterium* and *Lactobacillus* genera of gut microbiota ferment oligosaccharides for use as an energy source. As a by-product of this fermentation, gut microbiota produce lactic acid and short-chain fatty acids (SCFAs), such as acetate, butyrate, and propionate.

Suitably the use of this third aspect increases butyrate production in the gut of a consumer of the dietary supplement compared to the normal level of butyrate production in said consumer, suitably compared to a placebo dietary supplement which does not contain the composition comprising oligosaccharide compounds. Suitably the use increases butyrate production in the gut of a consumer of the dietary supplement compared to an alternative composition comprising oligosaccharide compounds which has a lower amount of components (a), (b) and/or (c) than specified herein.

Suitably the use of this third aspect promotes the growth of beneficial bacteria such as *Bifidobacterium* and *Lactobacillus* in the gut of the consumer which in turn may lead to the increase in butyrate production discussed above.

According to a fourth aspect of the present invention, there is provided a composition according to the first aspect or a pharmaceutical or nutraceutical composition according to the second aspect, for use as a medicament.

The composition for use of this fourth aspect may provide the increase in butyrate production discussed above in relation to the third aspect.

Although the effects of butyrate in the gut are not fully understood, it is believed that butyrate has several beneficial effects on human health including maintaining immune homeostasis, regulating systemic inflammation, regulating local and/or organ-specific inflammation including gut inflammation and provides the main energy source for enterocyte regeneration. Furthermore, butyrate may induce immune tolerance and therefore such an increased production of butyrate may be useful for the management and/or treatment and/or alleviation of the symptoms and/or alleviation of side effects of current treatment regimens of allergic diseases or other immune hypersensitivities, auto-immune diseases, stem cell transplantation (see "Short chain fatty acids as potential therapeutic agents in human gastrointestinal and inflammatory disorders", Gill PA, van Zelm MC, Muir JG, Gibson PR., Aliment Pharmacol Ther. 2018, 48(1), pages 15-34), graft-vs-host disease (see "The microbe-derived short-chain fatty acids butyrate and propionate are associated with protection from chronic GVHD", Markey KA, et al., Blood 2020, 136(1), pages 130-136) and cancer (see "The Clinical Link between Human Intestinal Microbiota and Systemic Cancer Therapy", Aarnoutse R, Ziemons J, Penders J, Rensen SS, de Vos-Geelen J, Smidt ML. Int J Mol Sci. 2019, 17, page 4145).

Therefore the fourth aspect of the present invention may provide the composition of the first aspect for use in the treatment management and/or treatment and/or alleviation of the symptoms of allergic diseases.

The fourth aspect of the present invention may provide the composition of the first aspect for use in the treatment management and/or treatment and/or alleviation of the symptoms of immune hypersensitivities.

The fourth aspect of the present invention may provide the composition of the first aspect for use in the treatment management and/or treatment and/or alleviation of the symptoms of auto-immune diseases.

The fourth aspect of the present invention may provide the composition of the first aspect for use in the treatment management and/or treatment and/or alleviation of the symptoms of stem cell transplantation.

The fourth aspect of the present invention may provide the composition of the first aspect for use in the treatment management and/or treatment and/or alleviation of the symptoms of graft-vs-host disease.

The fourth aspect of the present invention may provide the composition of the first aspect for use in the treatment management and/or treatment and/or alleviation of the symptoms of cancer.

Butyrate is also believed to play a role in the maintenance of the colonic epithelium; in regulating glucose homeostasis, lipid metabolism, and appetite regulation; and in regulating the immune system and inflammatory response (as described in Morrison, D.J.; Preston, T. "Formation of short chain fatty acids by the gut microbiota and their impact on human metabolism", Gut Microbes 2016, 7, 189-200). Therefore increasing butyrate production in the gut using the composition of the present invention is believed to provide the consumer with several such health benefits.

Suitably the composition for use of this fourth aspect promotes the growth of bifidobacteria in the gut of the subject.

The composition of the first aspect is a composition comprising oligosaccharide compounds, as described above. The composition comprising oligosaccharide compounds may have any of the suitably features and advantages described above in relation to the first aspect.

The use as a medicament of this fourth aspect may involve preventing the adhesion of pathogens or toxins produced by pathogens to the gut wall of a subject. The composition may be administered to a patient following a course of antibiotic treatment and/or chemotherapy and/or radiotherapy, which often alters or even destroys the normal healthy gut microbiota, or following surgery on the gut, in order to re-establish in the gut the normal microbiota of a healthy gut. The composition may be used in combination with one or more species of beneficial gut microbiota, suitably selected from the group of genus *Akkermansia, Bacteroides, Bifidobacterium, Lactobacillus* and *Parabacteroides,* for example *Bifidobacterium animalis, Bifidobacterium adolescentis, Bifidobacterium bifidum, Bifidobacterium breve, Bifidobacterium longum (includes B. infantis) and*/*or Megamonas.*

In the uses of the third and fourth aspects, the composition may modulate the abundance of said beneficial gut microbiota.

In the uses of the third and fourth aspects, the composition may modulate the abundance of a bacterial genus present in the GI tract. In some embodiments, the composition modulates the abundance of a bacterial genus present in one or both of the small intestine or large intestine. In some embodiments, the composition modulates the abundance of a bacterial genus predominant in the small intestine selected from the group of genus *Achromobacter, Agrobacterium, Blautia, Burkholderia, Coprococcus, Cryocola, Enterococcus, Eubacterium, Holdemania, Lactococcus, Mycobacterium, Pseudoramibacter, Ralstonia, Sphingomonas, Streptococcus,* and *Turicibacter.* In some embodiments, the composition modulates the abundance of a bacterial genus predominant in the large intestine selected from the group of genus *Anaerotruncus, Akkermansia, Bacteroides, Bilophila, Butyricimonas, Odoribacter, Parabacteroides, Phascolarctobacterium, Prevotella,* and *Ruminococcus.*

A method of preparing a composition comprising oligosaccharide compounds according to the first aspect, comprises the steps of:
(i) providing a source of saccharide compounds;
(ii) treating the source of saccharide compounds with one or more galactosidase enzyme to at least partially convert the source of saccharide compounds to the oligosaccharide compounds.

Suitably the steps of the method are carried out in the order step (i) followed by step (ii).

Suitably the source of saccharide compounds comprises lactose, lactulose or epilactose. Suitably the source of saccharide compounds comprises lactose. The source of saccharide may be lactose, for example a lactose syrup which may be derived from cow's milk. The lactose may be heat treated.

Optionally, no further saccharides, such as monosaccharides or disaccharides are added to the source of saccharide. In such cases the method produces galactooligosaccharide compounds.

Optionally, the source of saccharide compounds comprises at least one additional saccharide. Suitably the at least one additional saccharide provides the oligosaccharide compounds with an alternative terminal monosaccharide unit, as discussed above. Suitably the at least one additional saccharide is a source of such a monosaccharide unit. The at least one additional saccharide which is a source of such a monosaccharide unit may be a monosaccharide or may be a higher sugar, such as a disaccharide. The at least one additional saccharide may be a source of a monosaccharide selected from glucose (Glc), fucose (Fuc), arabinose (Ara), xylose (Xyl), rhamnose (Rha), mannose (Man), galactose (Gal), ribose (Rib), lyxose (Lyx), allose (All), altrose (Alt), gulose (Gul), idose (Ido), talose (Tal), psicose (Psi), fructose (Fru), sorbose (Sor), tagatose (Tag), galactosamine (GalN), glucosamine (GlcN) and N-Acetylglucosamine (GlcNAc) or a mixture thereof. The at least one additional saccharide may be one or more of the monosaccharides listed above.

In this case the method produces oligosaccharides having one or more of the above monosaccharides as the terminal sugar unit.

The at least one additional saccharide may be selected from Fucose, Arabinose, Xylose, Rhamnose, Mannose or mixtures thereof. The source of saccharides may comprise lactose and one or more sources of said monosaccharides. In this case the method produces oligosaccharides having as a terminal sugar unit selected from Glc, Fuc, Ara, Xyl, Rha and Man, or mixtures thereof.

Step (ii) of the method involves treating the source of saccharide compounds with at least one galactosidase enzyme. The galactosidase enzyme may be an alpha- or beta-galactosidase enzyme, depending on whether alpha or beta linkages between the saccharide units of the oligosaccharide compounds is required. Suitably the enzyme exhibits galactosyltransferase (transgalactosidic) activity and forms alpha- or beta- linkages between sugar units in the source of saccharide compounds. This results in the synthesis of oligosaccharide compounds with two or more galactose units derived from lactose. Suitably step (ii) is carried out until conversion of the source of saccharides to the oligosaccharide compounds is complete.

Step (ii) may involve treating the source of saccharide compounds with one or more additional enzymes which are not galactosidase enzymes.

Suitably the method comprises a step (iii) of separating the galactosidase enzyme from the composition comprising oligosaccharide compounds. Step (iii) may involve removing the enzyme by filtration, for example by nanofiltration.

The composition comprising oligosaccharide compounds produced in step (iii) may be heat treated.

Optionally, the composition is evaporated to reduce the water content to provide the final composition comprising oligosaccharide compounds as a syrup, as discussed above in relation to the first aspect.

Optionally, glucose is removed from the composition produced by step (iii) before evaporation. This suitably lowers the glucose content of the composition from 20-30 wt% to below 10 wt%, suitably approximately 5 wt% or lower. The water content of the composition is then reduced by evaporation and the product dried to provide the final composition comprising oligosaccharide compounds as a powder, as discussed above in relation to the first aspect.

It will also be appreciated that a person skilled in the relevant art could produce a composition comprising oligosaccharide compounds according to the first aspect, i.e. containing the specified amounts of particular oligosaccharide compounds, by combining said oligosaccharides obtained and isolated from different sources in the required amounts.

### Examples

### Example 1 - oligosaccharide syrup

Example 1 is a composition comprising oligosaccharide compounds, according to the present invention, in the form of a syrup. Such compositions may be obtained by the following procedure, which does not form part of the present invention. Lactose was rehydrated with potable water to give a working solution of between 35-65 wt% solids. The lactose solution was heat treated then cooled to 40-65°C. The pH of the solution was adjusted to pH 5.5-7.5. A beta-galactosidase enzyme was then added to the solution in a closed vessel and subsequently allowed to react with the lactose to catalyse the transfer of galactose molecules to produce oligosaccharide compounds. The progress of the reaction was monitored by measuring the generation of glucose. The reaction was allowed to proceed for between 8 and 26 hours. The reaction was then terminated by high heat treatment. The reaction mixture was cooled and filtered by carbon filtration to remove the enzyme. The mixture was then dried by evaporation to reduce the water content to approximately 22-28 wt% to provide the product as a syrup.

### Example 2 - oligosaccharide powder

Example 2 is a composition comprising oligosaccharide compounds, according to the present invention, in the form of a powder., Such compositions may be obtained by a modification of the procedure described above, which does not form part of the present invention. After removal of the enzyme, the mixture was further filtered to remove a significant portion of the glucose and other monosaccharides, reducing the monosaccharide content from around 23 wt% to around 5 wt%. The water content of the composition was then reduced by evaporation and the product dried to provide a powder having a water content to approximately 3-6 wt%.

### Example 3 - fractionation

Isolation of the different fractions (DP2, DP3, DP4 or DP5) from a sample of Example 2 was performed using a 5 × 70 cm BioGel P2 column, using water as eluent. The column was operated with a flow speed of 40 to 100 mL/h at room temperature (21 °C). Depending on the run, 0.5, 0.75, 1.0, 1.5 or 2.0 mL of a 0.5 g/mL solution of Example 2 in ultrapure water was loaded. The column was loaded in total 15 times with this solution to obtain enough material of the low abundant fractions, i.e. DP5. After a void volume of around 725 mL, 5 mL fractions were collected. Analysis using thin layer chromatography (TLC) and HPAEC-PAD were done to ensure molecules with the same DP are pooled.

Pooled fractions were frozen and lyophilized. The dry material of all runs was combined and redissolved. After a second cycle of freezing and lyophilization, the dry material was stored at 4 °C for further analyses or experimentation.

### Comparative Example 1

A commercially available composition comprising oligosaccharide compounds was obtained in powder form.

### Oligosaccharide analysis

Samples of Example 2 of the present invention and Comparative Example 1 were analysed to determine their oligosaccharide content by the following procedure.

### Materials and Methods

Samples of dry powder of each of Example 2 and Comparative Example 1 were dissolved in water to provide solutions having a concentration of 40 g/l for analysis.

### Gel permeation chromatography

An HPLC apparatus equipped with a Rezex RSO and a RI detector and in-line desalting (for removal of salts and charged material like proteins) was used for the aqueous GPC separation of the components of the samples. The separation was performed at elevated temperature (80°C). The separation range of the Rezex RSO column is from DP1 (monosaccharide) up to about DP10. All samples were analysed undiluted (at 40 g/L). Before analysis, all sample solutions were treated at 100°C for ten minutes in order to remove any microbiological or enzymatic activity.

### GOS fingerprinting

HPAEC-PAD (high performance anion exchange chromatography) equipped with a PA-1 column was used for separation of mono- and oligosaccharides of the different samples. Efforts were made to achieve a separation quality described in van Leeuwen et al., Carbohydrate Research 2016, 425, 48-58. A commercial maltooligosaccharide mixture and Comparative Example 1 were also injected for comparison of chromatograms with those reported by Van Leeuwen et al. Based on this, peak annotations were made for a number of peaks. The samples as used for GPC were 100-fold diluted with DMSO before injection.

### Results

### Gel permeation chromatography (GPC-RI)

Table 1 shows DP (degree of polymerisation) composition results for the samples using Rezex-RSO system, based on RI calibration with glucose (values expressed as g/L in the samples). All material eluting in the >DP5 window was combined.

**Table 1**

| | Comp. Ex. 1 | | Example 2 | |
|---|---|---|---|---|
| Fraction | Conc. (g/l) | wt% | Conc. (g/l) | wt% |
| DP>5 | 1.1 | 2.8 | 1.6 | 4.2 |
| DP=5 | 2.2 | 5.6 | 2.6 | 6.9 |
| DP=4 | 5.1 | 13.1 | 6.1 | 16.1 |
| DP=3 | 10.7 | 27.4 | 12.6 | 33.2 |
| DP=2 | 17.8 | 45.6 | 13.1 | 34.6 |
| glucose | 2.1 | 5.4 | 1.7 | 4.5 |
| galactose | 0.1 | 0.3 | 0.2 | 0.5 |
| Total | 39.0 | 100.0 | 37.9 | 100.0 |
| Total DP 2-5 | 35.7 | | 34.4 | |

The concentration information can be used to calculate the relative weight percentages of the different DP fractions of oligosaccharides contained in the samples, as shown in Table 1, wherein DP=2 refers to disaccharides, DP=3 refers to trisaccharides etc.

### GOS fingerprinting

To identify the individual galactooligosaccharides in the sample (GOS fingerprinting) a gradient was developed giving comparable separation to that reported in van Leeuwen et al., Carbohydrate Research 2016, 425, 48-58. Peak annotations were made in the chromatograms of all GOS samples based on peak annotations made in van Leeuwen et al. for the oligosaccharide compounds. Retention windows of about 15 seconds were applied for peak annotation.

Table 2 shows information on HPAEC-PAD peak areas of all annotated peaks together with information on incubation conditions, sample concentration, dilution and injection volume as shown.

**Table 2**

| | Comp. Ex. 1 | | Example 2 | |
|---|---|---|---|---|
| Compound ID | Peak areas | wt% | Peak areas | wt% |
| Unknown 1 | 1.6 | 0.6 | 0.5 | 0.2 |
| Unknown 2 | 1.5 | 0.5 | 1.9 | 0.7 |
| Galactose | 0.5 | 0.2 | 1.3 | 0.5 |
| Glucose | 19.5 | 7.0 | 17.5 | 6.7 |
| Unknown DP1 | 0.6 | 0.2 | 1.8 | 0.7 |
| Unknown 3 | 1.5 | 0.5 | 1.2 | 0.5 |
| 3 | 0.2 | 0.1 | 0.9 | 0.3 |
| Allolactose | 16.2 | 5.8 | 24 | 9.1 |
| Lactose | 48.4 | 17.5 | 30.9 | 11.8 |
| 6 | 7.2 | 2.6 | 2.1 | 0.8 |
| Unknown DP3 | 3.5 | 1.3 | 0.8 | 0.3 |
| 8a | 48.5 | 17.5 | 38.5 | 14.7 |
| 8b | 18.1 | 6.5 | 14.6 | 5.6 |
| 9 | 7.9 | 2.9 | 8.1 | 3.1 |
| 10 | 16 | 5.8 | 13.1 | 5.0 |
| 11 | 27.6 | 10.0 | 20.4 | 7.8 |
| 12 | 1.4 | 0.5 | 28.9 | 11.0 |
| 13 | 20.4 | 7.4 | 4.3 | 1.6 |
| 17 | 11.8 | 4.3 | 2.7 | 1.0 |
| 18 | 7.1 | 2.6 | 0.9 | 0.3 |
| 22 | 6.4 | 2.3 | 3.9 | 1.5 |
| 23 | 1.9 | 0.7 | 4.6 | 1.8 |
| 24 | 1.1 | 0.4 | 2 | 0.8 |
| 29 | 4.7 | 1.7 | 12.8 | 4.9 |
| 30 | 2.6 | 0.9 | 12.6 | 4.8 |
| 31 | 0.8 | 0.3 | 12.3 | 4.7 |
| sum | 277.1 | 100.0 | 262.5 | 100.0 |

The peak areas were assumed to approximately correspond to the amount of each oligosaccharide compound present in the composition. Where a particular oligosaccharide compound was not identified then "unknown" and a number is entered in the table. The identified compounds are either identified by name or by a number which corresponds to the number assigned to particular galactooligosaccharides in van Leeuwen et al., Carbohydrate Research 2016, 425, 48-58. A list of these galactooligosaccharides and their corresponding numbers is provided below.

The oligosaccharide components (a)-(e) discussed above, wherein each X group is Glu, correspond to the following numbered entries in Table 2 above:
(a) D-Gal-(β1-3)-D-Gal-(β1-4)-D-Glu - **12**
(b) D-Gal-(β1-3)-D-Gal-(β1-3)-D-Glu - **29**
(c) D-Gal-(β1-3)-D-Gal-(β1-2)-D-Glu - **30**
(d) D-Gal-(β1-3)-D-Gal-(β1-3)-D-Gal-(β1-4)-D-Glu - **31**
(e) D-Gal-(β1-4)-D-Gal-(β1-4)-D-Glu - **11.**

### Prebiotic effects - butyrate production

The prebiotic effects of the compositions of the present invention were investigated and compared to a comparative GOS composition using the following procedure.

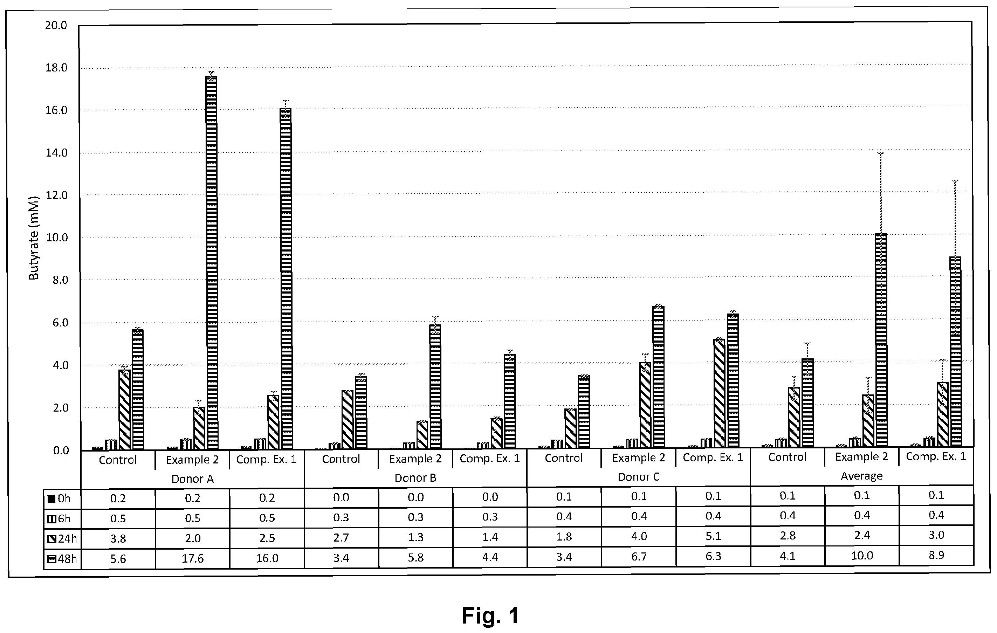

The compositions of Example 2 of the present invention, Comparative Example 1 and a control blank sample were subjected to dialysis using a 0.5 kDa membrane to provide 5 g/l samples which were then mixed with faecal matter obtained from three healthy human adult subjects (donors A, B and C). The mixtures were shaken under anaerobic conditions and monitored over a 48 hour period for colonic fermentation products including butyrate (with 6, 24 and 48 h collection points). The distribution of oligosaccharides in the mixtures was also monitored over this time period using the method described above in relation to Table 2. The results show that Example 2 was well fermented by all donors, mainly during the time period 0-24 hours and that butyrate production increased compared to the Comparative Example 1 and the control at the 6 and 48 hour time points. The results of the butyrate analysis are shown in Figure 1. The results of the oligosaccharides analysis for the samples at the different time points are shown in Table 3. These results show that the oligosaccharides in the samples were actively consumed by the microbiota present in the faecal samples during the experiments.

To assess whether treatment effects on gut microbial activity were statistically significant, three two-sided T-tests were performed between Example 2 and the control, Comparative Example 1 and the control and Example 2 and Comparative Example 1 to obtain p-values. The Benjamini-Hochberg false discovery rate (FDR) was also used in this analysis. Differences between treatment effects were considered significant when the obtained p-value was smaller than a reference value. Table 4 below shows the differences in the averaged butyrate production for the compared samples over 48 hours and the asterisk denotes whether the difference was considered significant according to the analysis described above. These results show that the increase in butyrate production provided by Example 2 during the time period 0-48 hours was statistically significant compared to the control and Comparative Example 1.

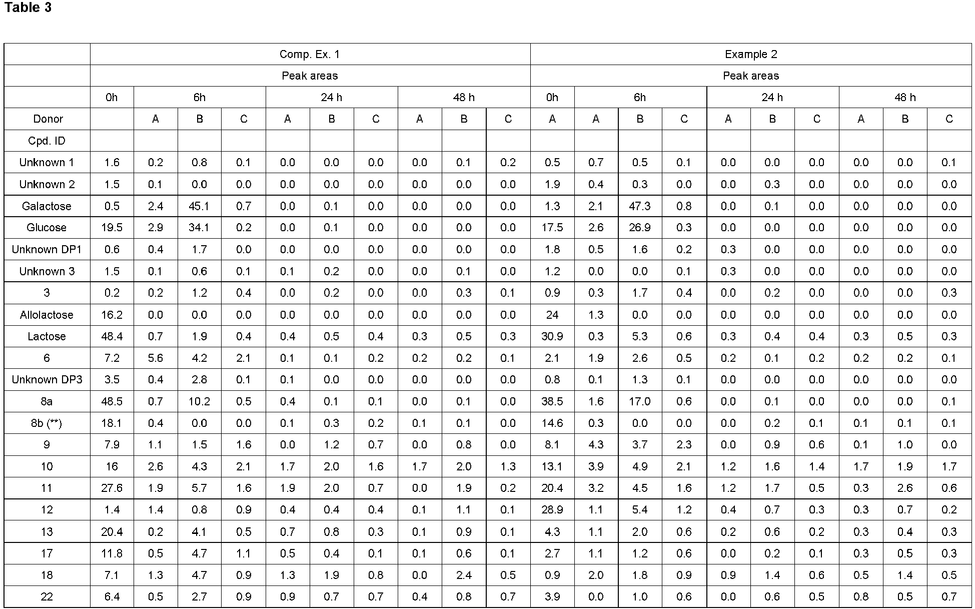

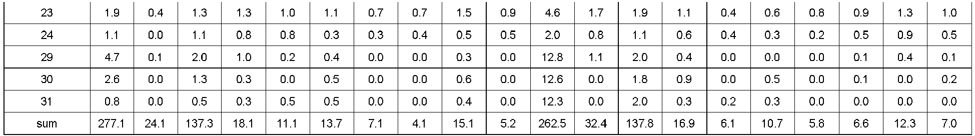

**Table 4**

| D0-48h / Production | Example 2 - Control | Comp. Ex. 1 - Control | Example 2 - Comp. Ex. 1 |
|---|---|---|---|
| Butyrate (mM) | 5.88* | 4.75* | 1.13* |

### Examples 4-9 - oligosaccharides with alternative terminal monosaccharides

Example compositions 4-9 comprising oligosaccharide compounds having alternative terminal monosaccharides (i.e. alternative to glucose) according to the present invention were produced by the procedure described above in relation to Example 1, using the substrates noted in Table 5 below - i.e. a combination of lactose and the additional substrate noted for Examples 4-8 and lactulose only for Example 9. Significantly higher molar excess of acceptor (substrate) was deliberately utilised to promote the formation of GOS compounds comprising the alternative terminal monosaccharides.

**Table 5**

| | Substrate (mg/ml) | | | | | | |
|---|---|---|---|---|---|---|---|
| Ex. | lactose | L-fucose | L-arabinose | D-xylose | L-rhamnose | sucrose | lactulose |
| 4 | 250 | 500 | - | - | - | - | - |
| 5 | 250 | - | 500 | - | - | - | - |
| 6 | 250 | - | - | 500 | - | - | - |
| 7 | 250 | - | - | - | 500 | - | - |
| 8 | 250 | - | - | - | - | 700 | - |
| 9 | - | - | - | - | - | - | 700 |

### Characterisation and quantification of Examples 4-9 using HPAEC-PAD

The GOS compositions of Examples 4-9 were separated and quantified by High-Performance Anion-Exchange Chromatography with Pulsed Amperometric Detection (HPAEC-PAD), using a CarboPac PA210 (2 x 150 mm) analytical column with a CarboPac-PA1 (4 x 50 mm) guard column. Operation parameters were set as follows: temperature of the column was 30°C; elution was performed using gradient concentrations of sodium hydroxide and sodium acetate at a flow rate of 0.2 ml/min. Eluents were as follows: A - Milli-Q water, B - 500 mM NaOH, C - 500 mM NaOH combined with 600 mM NaOAc, D - Milli-Q water. DP1, DP2 and higher order GOS peaks were quantified based on calculating the peak areas and employing constructed calibration curves using available standards. Glucose, galactose and lactose were calibrated in the 5 - 300 µg/ml range. Novel GOS DP2 peaks were quantified based on lactose calibration. Novel GOS, DP3 and DP4 peaks, were quantified in reference to an external malto-oligosaccharide ladder (DP1-8).

### Purification of GOS on activated charcoal

In order to remove monosaccharides, the GOS compositions were purified with activated charcoal following methodology described by Julio-González et al. (Food Research International, Vol. 129, March 2020, 108811) with some modifications. GOS reaction mixtures were combined with activated charcoal using the ratio: 6g of charcoal for 0.5g total sugar. The ratio was adjusted to the final concentration with ethanol (either 1%, 3% or 5% v/v) for monosaccharide desorption. The resulting mixture was stirred for 30 min, 2h, 4h and overnight at 25 °C to determine an optimal desorption time, and then filtered through Whatman No.1 paper (Whatman International Ltd., Maidstone, UK) under vacuum (step 1). This process was repeated for a second time to ensure removal of total monosaccharides (step 2). Desorption of oligosaccharides from washed activated charcoal was carried out by adding an appropriate volume of ethanol (50%, v/v) and stirring the mixture for 120 min followed by filtration (step 3). The resultant GOS suspension in ethanol was freeze-dried to obtain the powder subsequently tested as microbial growth substrates in fermentation assessments.

The HPAEC-PAD chromatography showed that Examples 4-9 contained different GOS compounds to Example 1, due to the incorporation in the compounds of the respective alternative terminal monosaccharides. The formation of these different GOS compounds in the respective product mixtures demonstrates that the beta-galactosidase enzyme can utilize alternative acceptor molecules in addition to lactose in order to provide GOS compounds with alternative terminal monosaccharides.

Table 6 shows the percentage quantity of different types of GOS compounds produced in Examples 4-9. The quantification of HPAEC-PAD peaks was based the calibration curves using available standards. *^{a}*Total GOS: all the quantified transgalactosylated carbohydrate peaks, identified in HPAEC-PAD, except remaining glucose, galactose and lactose. *^{b}*Total GOS (-acceptor): equivalent to previous, except excluding the remaining quantity of acceptor molecule in the calculation. *^{c}*nGOS: all the quantified emerging novel peaks in chromatograms of lactose/novel acceptor reactions, also absent in lactose-only Example 1 reaction - disregarding purely lactose-derived peaks as evident in Example 1 chromatogram. *^{d}*nGOS (-acceptor): equivalent to previous, except excluding the remaining quantity of acceptor molecule in the calculation. *^{e}*nGOS in total GOS: percentage of quantified novel GOS peaks from total GOS produced in each reaction - to distinguish from purely lactose-derived GOS quantities. *^{f}*All the reactions were terminated at the optimal 24h timepoint, except LactulOS (highest yield of novel fructosylated GOS observed at 12h). The standard deviation was derived from two independent experiments (provided in brackets).

**Table 6**

| Example | Total GOS*^{a}* | | Total GOS*^{b}* (-acceptor) | | nGOS*^{c}* | | nGOS*^{d}* (-acceptor) | | nGOS*^{e}* in total GOS | |
|---|---|---|---|---|---|---|---|---|---|---|
| 4 | 20.3 | (1.9) | 60.9 | (2.2) | 5.0 | (0.8) | 15.1 | (1.5) | 24.7 | (1.6) |
| 5 | 22.8 | (1.6) | 56.2 | (3.3) | 14.9 | (1.5) | 36.7 | (3.4) | 65.2 | (2.2) |
| 6 | 32.6 | (3.4) | 67.3 | (1.1) | 5.7 | (1.3) | 11.7 | (1.6) | 17.3 | (2.1) |
| 7 | 28.3 | (2.1) | 54.9 | (0.5) | 18.5 | (1.8) | 35.8 | (1.1) | 65.2 | (1.4) |
| 8 | 58.2 | (3.9) | 69.2 | (3.5) | 25.6 | (3.0) | 30.3 | (3.2) | 43.8 | (2.3) |
| 9 | 42.2 | (1.1) | - | - | - | - | - | - | - | - |

The best yields from were obtained with Example 8 (58.2%) and Example 9 (42.2%). The other examples exhibited markedly lower yields (20 - 30 %) compared to Example 1, when calculating against total sugar in the reaction. However, adjusting the same yields to the actual transgalactosylated sugar present in the final matrix, by removing the excess of acceptor molecule quantified weight, provides a clearer result (Table 6, 2nd column). Total GOS (-acceptor) reaction yields in Examples 4-8 were comparable (55 - 70%) to the overall GOS yields observed in Examples 1 and 9. The most abundant formation of novel hybrid transgalactosylated acceptor species was observed in Example 5 (36.7%) and Example 7 (35.8%) (Table 6, 4th column), suggesting that L-arabinose and L-rhamnose are the best alternative acceptor substrates for the enzyme.

Following purification using activated charcoal, as described above, the relative abundance of monosaccharide was greatly reduced to almost equivalent levels in all carbohydrate mixtures. The remaining quantity of monosaccharide from total carbohydrate in all preparations barely exceeded 5% levels following this purification step (Table 7). In this way, the relative abundances of transgalactosylated oligosaccharides in the obtained mixtures were significantly elevated, concentrating more important polymeric GOS carbohydrate molecules. In terms of the subsequent *in vitro* fermentation trials, elimination of simple sugars was vital in order to minimise the effects on monosaccharide fermentation in the initial stages (< 8h) after faecal inoculation. Instead, the obvious aim of such fermentation simulations is to establish the genuine differences in fermentation profiles among newly synthesized GOS varieties, particularly at a higher DP range.

**Table 7**

| Example | MONO_{before} | MONO_{after} | Total GOS_{before} | Total GOS_{after} |
|---|---|---|---|---|
| 4 | 74.5 | 4.8 | 20.3 | 85.0 |
| 5 | 71.5 | 5.5 | 22.8 | 83.8 |
| 6 | 63.3 | 4.2 | 32.6 | 86.7 |
| 7 | 65.8 | 5.4 | 28.3 | 83.7 |
| 8 | 23.3 | 3.2 | 58.2 | 73.3 |
| 9 | 29.4 | 5.2 | 42.2 | 61.4 |

### Examples 4-9 - Faecal fermentation studies

### Faecal Sample Preparation

Fresh faecal samples were collected 1 h prior to inoculation from three adult male and female healthy donors, who had not taken antibiotics for 3 months beforehand and had no history of gastrointestinal disorder. Anaerobic conditions were maintained in the collection containers using sachets that absorb atmospheric oxygen in the jar, whilst simultaneously liberating carbon dioxide, hydrogen and nitrogen (Thermo Scientific^{™}, Basingstoke, UK, Oxoid AnaeroGen 2.5 L). The faecal slurry was prepared by diluting the stool in phosphate saline buffer (PBS, pH 7.0) at a ratio of 1:10 and mixed in a stomacher for 2 min. Batch culture experiments were performed in duplicate for each donor.

### Batch Culture Fermentations

The method used was as described by Olano-Martin et al. 2000 ('In vitro fermentability of dextran, oligodextran and maltodextrin by human gut bacteria.' British Journal of Nutrition, 83(3), 2000, p.247-255). 9 mL of autoclaved nutrient medium was added to 10 mL working volume fermentation vessels. This medium contained (g/L⁻¹): peptone water, 2; yeast extract, 2; NaCl, 0.1; K₂HPO₄, 0.04; KH₂PO₄, 0.04; MgSO₄.7H₂O, 0.01; CaCl₂.6H₂O, 0.01; NaHCO₃, 2; hemin (dissolved in a few drops of 1 mol 1⁻¹ NaOH), 0.05; cysteine HCl, 0.5; bile salts, 0.5; Tween 80, 2 and 10 µL vitamin K topped up to 1 L with ddH₂0. The medium was flushed with N₂ overnight to create anaerobic conditions. Vessels were maintained at 37 °C via a circulating water bath and pH was maintained between 6.7 and 6.9 to mimic conditions in the distal colon, using a pH controller connected to 0.25 M solutions of HCl and NaOH (Electrolab, Tewkesbury, UK). Immediately prior to faecal sample inoculation, 0.1 g of test substrates (1%, w/v) were added to the vessels before adding 1 mL (v/v) faecal slurry (Table 1). Samples were removed from the fermenters immediately after inoculation of the slurry (0 h) and 8 h and 24 h for enumeration of bacteria and metabolite analyses. The fermentation experiments were performed in duplicate for each substrate using faecal inocula from three adult volunteers.

### Preparation of the Samples for Enumeration of Bacteria by Flow Cytometry-Fluorescent In Situ Hybridisation (FISH)

Samples (750 µL) were removed from *in vitro* fermentation vessels at 0, 8 and 24 h and immediately placed on ice, before centrifugation at 13,000× g for 5 min and the supernatant discarded. Pelleted bacteria were fixed for 4 h at 4 °C in PBS and 4% (w/v) filtered paraformaldehyde (PFA, pH 7.2) in a ratio of 1:3 (v/v). Samples were washed twice with filtered PBS and resuspended in 300 µL of PBS and ethanol (1:1, v/v) then stored at - 20 °C for up to 3 months.

### Flow- FISH Analysis

Hybridisation was carried out using a method as described by Rigottier-Gois et al. 2003 ('Fluorescent hybridisation combined with flow cytometry and hybridisation of total RNA to analyse the composition of microbial communities in human faeces using 16S rRNA probes.' FEMS Microbiology Ecology, 43(2), 2003, p.237-245), using genus and group specific 16S rRNA-targeted oligonucleotide probes (Eurofins Genomics, Wolverhampton, UK). Primers used were the commercially available Ato291, Bac303, Bif164, Chis150, DSV687, Erec482, Lab158, Rrec584, Fprau655, Prop853 and Eub338. Samples were screened using a flow cytometer (Accuri C6, BD Biosciences, USA) with Accuri CFlow software.

Once defrosted, samples were then vortexed for 10 s and 75 µL of the sample added to 500 µL of PBS in a 1.5 mL tube to be vortexed again and centrifuged at 13,000x g for 3 min. The supernatant was removed and discarded. 100 µL Tris-EDTA buffer containing lysozyme (1 mg/mL) was added to the tube, mixed using a pipette, then incubated in the dark for 10 min. Samples were vortexed and centrifuged at 13,000x g for 3 min and the supernatant removed. 500 µL of PBS was added to the tube, the pellet resuspended using a pipette then vortexed and centrifuged at 13,000x g for 3 mins. Supernatant was removed and pellets resuspended in 150 µL of hybridisation buffer (0.9 M NaCl, 0.2 M Tris-HCl (pH 8.0), 0.01% sodium dodecyl sulphate, 30% formamide), vortexed and centrifuged at 13,000× g for 3 min. Supernatant was again removed and pellets resuspended in 1 mL hybridisation buffer. 4 µL of oligonucleotide probe solutions (50 ng/uL) (see Supplementary Table 1 were added to a 1.5 mL centrifuge tube with 50 µL of the sample, vortexed and incubated at 36 ∘C overnight. Once hybridisation had been completed, 125 µL of hybridisation buffer was added to each tube, vortexed, centrifuged at 13,000x g for 3 mins and the supernatant carefully removed. 175 µL of washing buffer (0.064 M NaCl, 0.02 M Tris/HCl (pH 8.0), 0.5 M EDTA (pH 8.0), 0.01% sodium dodecyl sulphate) kept at 40 ∘C was added to each tube, resuspending the pellets. The tubes were then vortexed and incubated for 20 min at 38 ∘C in the dark, to remove non-specific binding of primers. Samples were then centrifuged at 13,000x g for 3 min, the supernatant removed, 300 µL PBS added, then vortexed. Samples were kept refrigerated at 4 ∘C in the dark prior to flow cytometry analysis.

### Gas Chromatography Mass Spectroscopy (GC-MS) for SCFA and Lactate Analysis

1.5 mL samples from each vessel were taken at 0, 8 and 24 h and centrifuged at 13,000× g for 10 min and supernatants stored at -20 °C until needed for metabolite analysis. Samples were prepared for GC- MS using the SCFA derivatisation method as described by Richardson et al., 1989 ('Simultaneous determination of volatile and non-volatile acidic fermentation products of anaerobes by capillary gas chromatography.' Letters in Applied Microbiology, 9(1), 1989, p.5-8). Once the samples had been removed from -20 °C storage and had defrosted they were vortexed and centrifuged again at 13,000× g for 10 min. 1 mL aliquots of sample supernatant were then transferred into flat-bottomed glass tubes with 50 µL internal standard solution (0.1 M 2-ethylbutyric acid). 500 µL of concentrated hydrochloric acid (HCl) and 3 mL of diethyl ether were added to the sample in each glass tube, which was then vortexed for 1 min and centrifuged at 2000x g for 10 min. 400 µL of pooled ether extract was then added to 50 µL N-(tert-butyldimethylsilyl)-N-methyltrifluoroacetamide (MTBSTFA) in a GC screw-cap vial. External samples of 1 M acetic, butyric, propionic and lactic acid were also prepared using Richardson SCFA derivatisation method. Samples were left at room temperature for 72 h to allow metabolites to completely derivatise. An Agilent/HP 6890 Gas Chromatograph (Hewlett Packard, UK) fitted with a HP-5MS 30 m × 0.25 mm column with a 0.25 µm coating (Crosslinked (5%-Phenyl)-methylpolysiloxane, Hewlett Packard, UK) was used for metabolite analysis. Quantification of the samples was obtained through calibration curves of lactic, acetic, propionic, butyric acids at concentrations between 6.25 and 100 mM from the external standards.

### Results - Bacterial populations

In the Figures and description which follows, Examples 4-9 are referred to as follows with reference to the additional substrate used in the production of these compositions:
Example 4 - FucOS
Example 5 - AraOS
Example 6 - XylOS
Example 7 - RhaOS
Example 8 - SucOS
Example 9 - LactulOS

The sample denoted B-GOS (galactose containing oligosaccharide) was produced using 500 mg/ml of lactose as substrate.

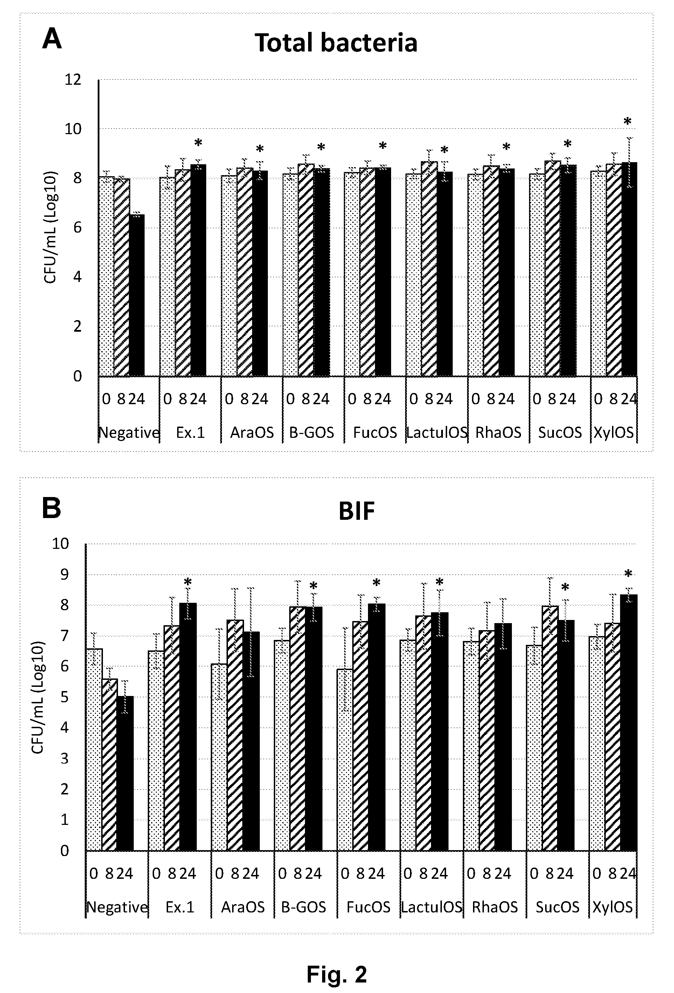

Differences in bacterial populations observed for Examples 4-9 are shown in Fig. 2. Figure 2 shows the effects of different novel oligosaccharides on bacterial counts (log colony-forming units (CFU/mL) of *Lactobacillus* species (LAB, **A**), *Bacteroidaceae* and *Prevotellaceae* (BAC, **B**), *Clostridium coccoides- Eubacterium rectale* group (EREC, **C**), *Roseburia* subcluster (RREC, **D**), *Atopobium-Coriobacterium* species (ATO, **E**), *Clostridium* cluster IX (PROP, F), *Faecalibacterium prausnitzii* (FPRAU, **G**), *Desulfovibrio* (DSV, **H**) and *Clostridium histolyticum* (CHIS, **I**) detected by flow-FISH in pH-controlled batch culture experiments. Samples were collected at 0, 8 and 24 h. Error bars are mean standard error. Significant differences (p≤0.05) are denoted by *.

Batch culture experiments were repeated using fresh stool samples from the same donors used in the first set of experiments. There were no significant differences between the bacterial populations in donors between the two technical repeats at all three sampling time points, 0, 8 or 24 h.

Figure 2 shows the effects of Examples 4-9 on total bacteria counts (**A**) and Bifidobacterium species (**B**) (log colony-forming units (CFU/mL) detected by flow-FISH cytometry in pH-controlled batch cultures. Samples were taken at 0, 8 and 24 h. One-Way ANOVA was applied to test the main interaction between groups. Error bars are mean standard error. Significant differences (p≤0.05 (F-test)) are denoted by *. The negative control has no added carbon source.

Increases in total bacterial numbers (Fig.2A) were observed after 8 h for all test oligosaccharides and positive controls, with statistically significant increases detected in Examples 4-9 (p ≤ 0.05) after 24 h fermentation. The greatest increase in total bacteria was seen in Example 6 at 24 h, with Log₁₀ 8.64 ± 0.99 CFU/mL.

Significant increases in *Bifidobacterium* species numbers (Fig. 2B) were observed for Example 1 (p= 0.013), Example 4 (FucOS) (p= 0.006), Example 9 (LactulOS) (p= 0.006), Example 8 (SucOS) (p= 0.042) and Example 6 (XylOS) (p= 0.040) after 24 h fermentation. The greatest percentage increase was 63% from 0 h to 24 h, which was observed with AraOS (Log₁₀ 6.08 (±1.14) to Log₁₀ 7.12 (±1.44).

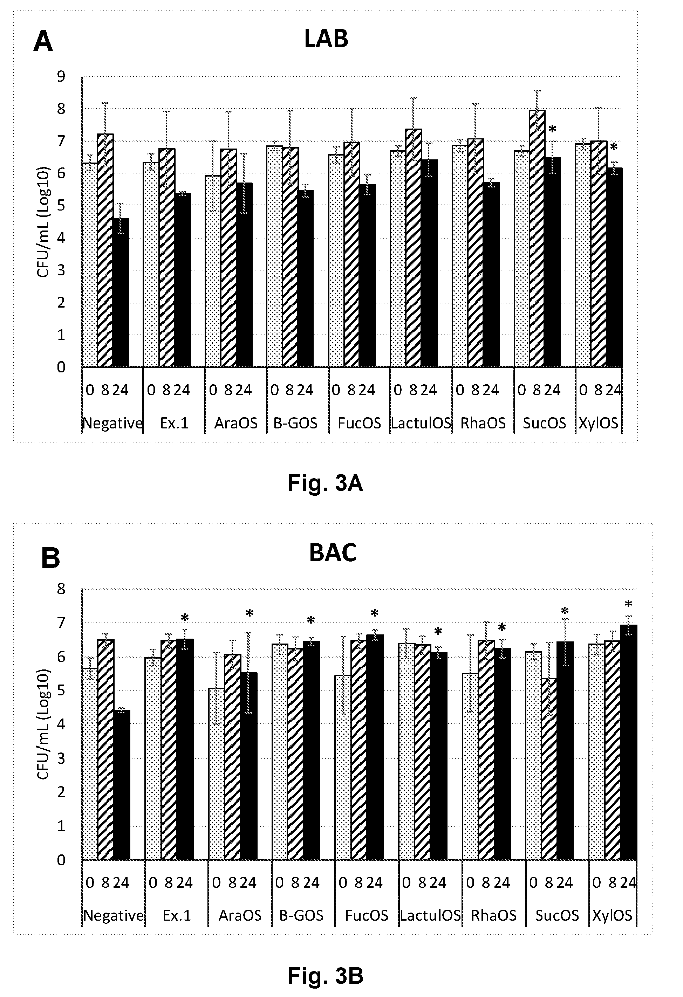

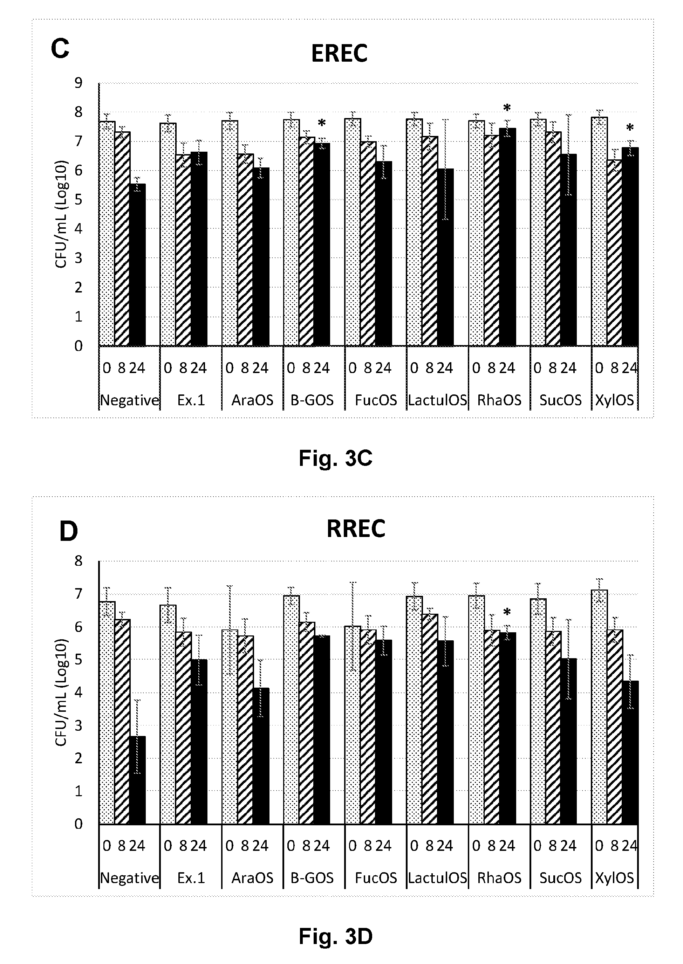

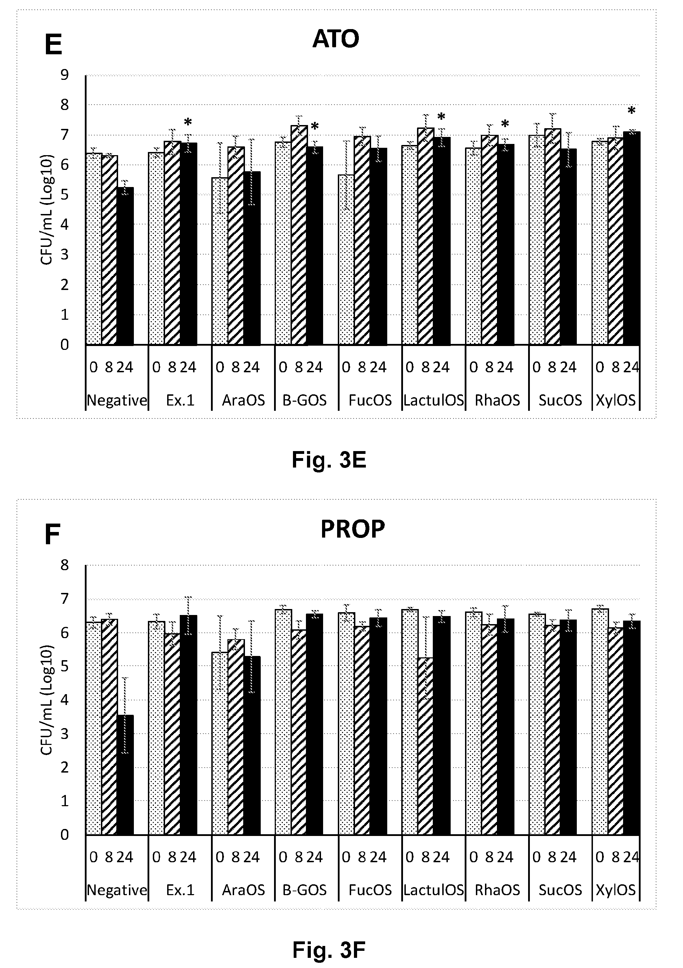

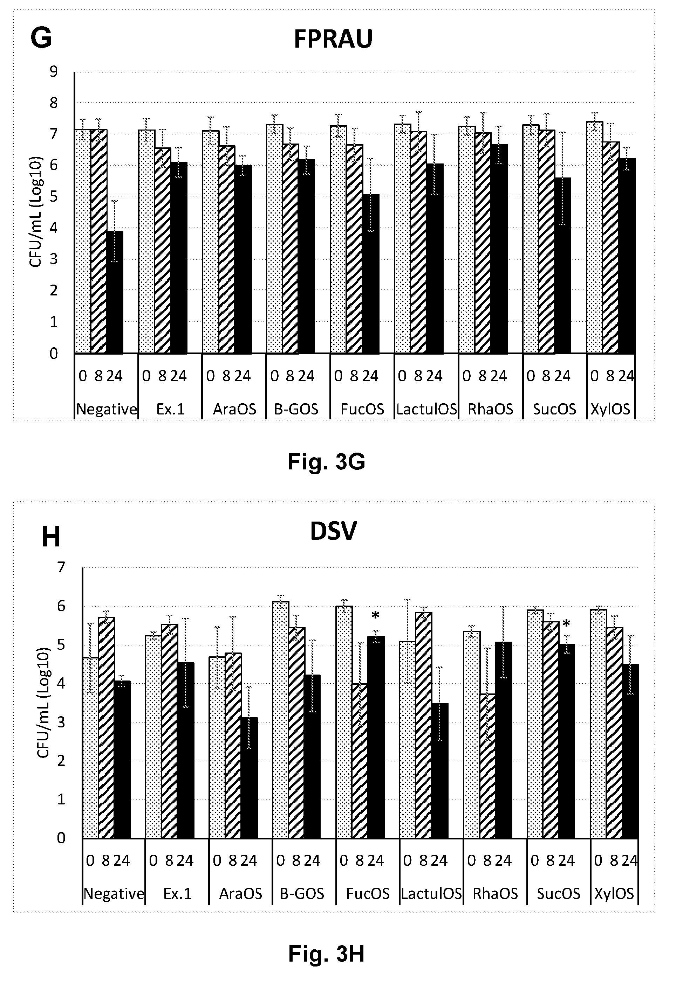

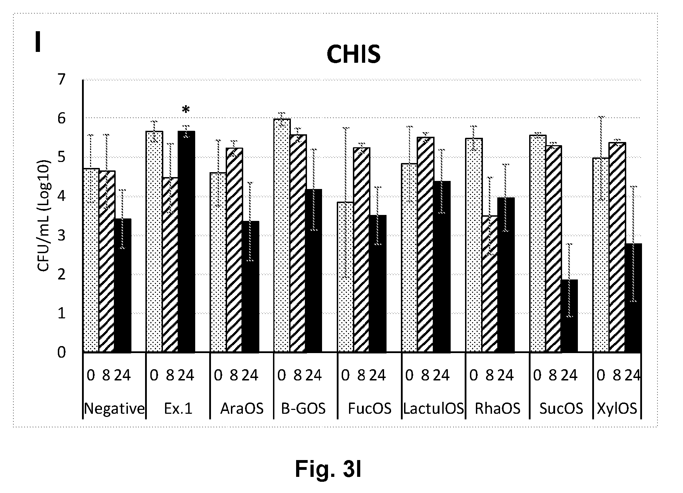

Figure 3 shows the effects of Examples 4-9 on bacterial counts (log colony-forming units (CFU/mL) of *Lactobacillus* species (LAB, **A**), *Bacteroidaceae* and *Prevotellaceae* (BAC, **B**), *Clostridium coccoides- Eubacterium rectale* group (EREC, **C**), *Roseburia* subcluster (RREC, **D**), *Atopobium-Coriobacterium* species (ATO, **E**), *Clostridium* cluster IX (PROP, **F**), *Faecalibacterium prausnitzii* (FPRAU, **G**), *Desulfovibrio* (DSV, **H**) and *Clostridium histolyticum* (CHIS, **I**) detected by flow-FISH in pH-controlled batch culture experiments. Samples were collected at 0, 8 and 24 h. Error bars are mean standard error. Significant differences (p≤0.05) are denoted by *. Again, the negative control has no added carbon source.

Significant differences in *Lactobacillus* numbers (Fig. 3A) were observed with Example 8 (SucOS) (p= 0.049) and Example 6 (XylOS) (p= 0.036) treatments following 24 h fermentation. In vessels treated with B-GOS, Example 7 (RhaOS) and Example 6 (XylOS) there were significant increases in *Clostridium coccoides- Eubacterium rectale* group, whilst the only treatment where a significant increase in *Roseburia* subcluster populations was observed was with Example 7 (RhaOS) (p= 0.05) at 24 h fermentation (Fig. 3D). There were no significant differences in *Clostridium* cluster IX (PROP), *Faecalibacterium prausnitzii* (FPRAU) or *Desulfovibrio* (DSV) bacterial populations regardless of treatments (Fig. 3F, 3G and 3H respectively).

There were significant increases in *Atopobium- Coriobacterium* (ATO) (Fig.3E) in vessels containing B-GOS, Example 9 (LactulOS), Example 7 (RhaOS) and Example 6 (XylOS). *Clostridium histolyticum* (CHIS) populations also increased significantly (Fig. 3I) at 24 h in the fermentation vessel treated with Bimuno^{®} (p= 0.042). However, *Clostridium histolyticum* population numbers (Log₁₀ 5.67 (±0.14), were considerably lower than the total bacteria (Fig. 3A) count at the same 24 h time point at Log₁₀ 8.56 ± (0.17) and levels were close to the limits of detection.

### Results - Bacterial metabolites

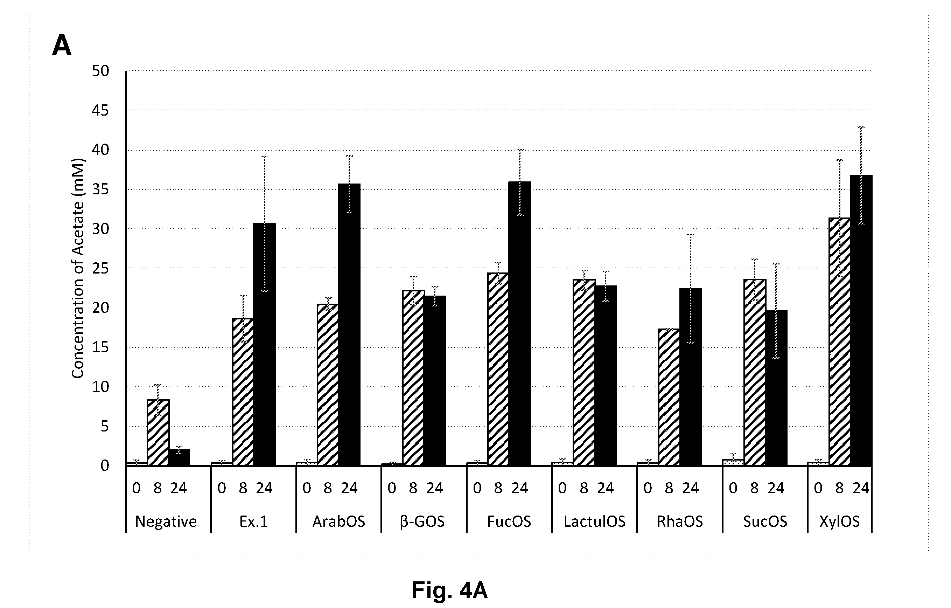

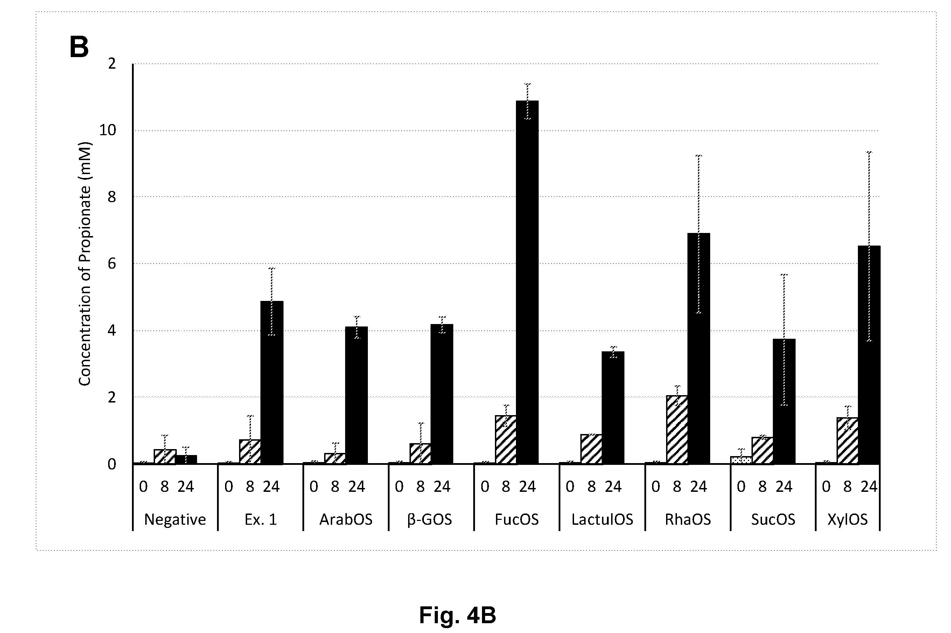

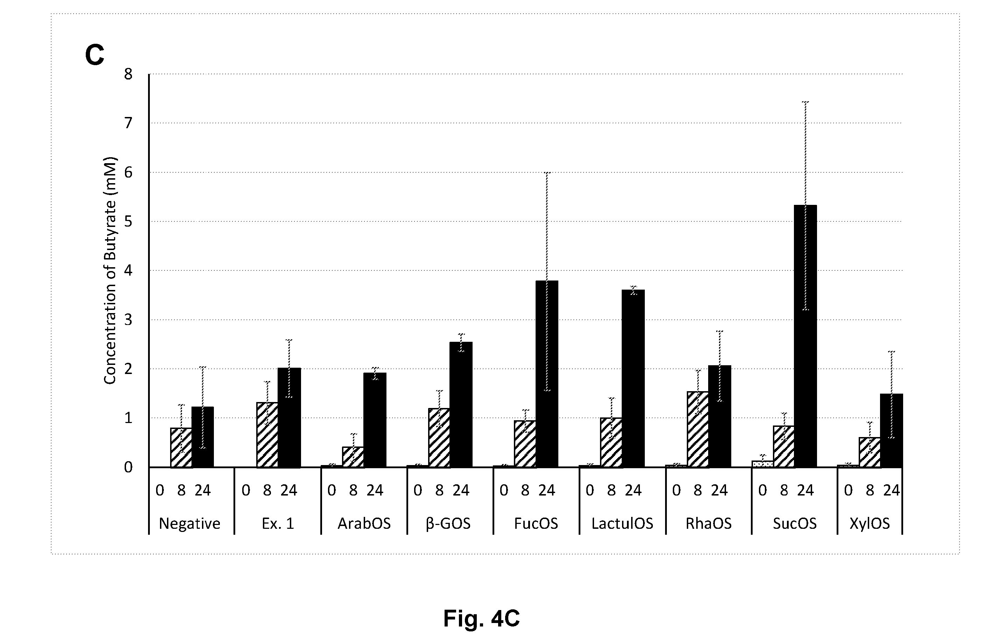

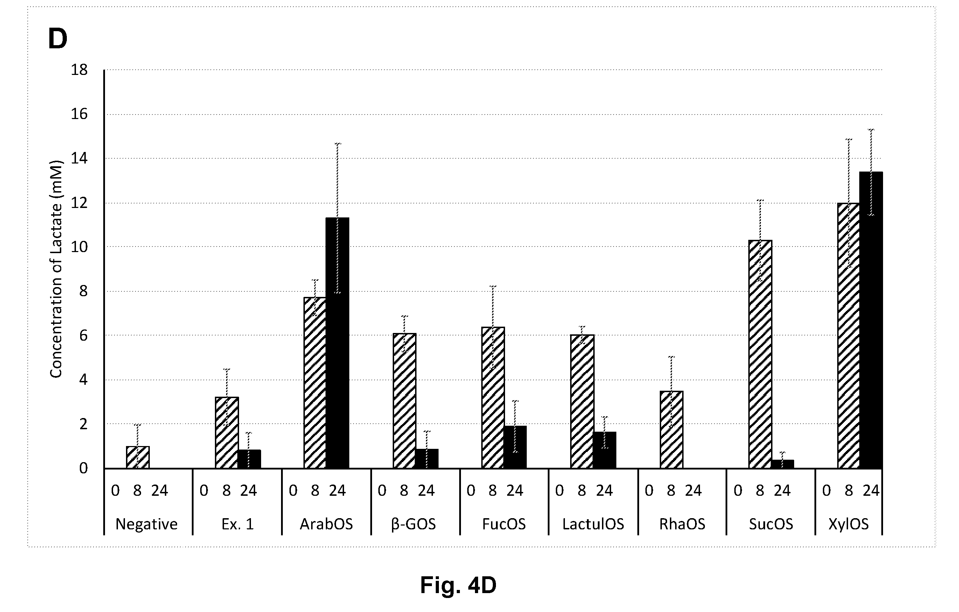

Figure 4 shows the concentration of bacterial metabolites: acetate (**A**), propionate (**B**), butyrate (**C**) and lactate (**D**) detected by GC-MS at 0, 8 and 24 h in samples taken from pH-controlled batch cultures testing newly synthesised oligosaccharides and controls. Error bars are mean standard error.

Concentrations of acetate, propionate, butyrate and lactate increased following 8 h and 24 h of fermentation when compared to 0 h for all Examples and positive controls tested in batch culture fermentations (Fig. 4). Acetate was the most prevalent SCFA produced in all the batch culture fermentations reaching the highest concentrations compared to other metabolites measured (Fig. 4A). The greatest increase in acetate was observed with Example 6 (XylOS) at 24 hours, rising to a concentration of 36.8 mM from 0.4 mM at 0 h. increases of acetate were also seen in the Example 1, Example 5 (AraOS) and Example 4 (FucOS) samples over 24 h of fermentation.

SCFAs propionate and butyrate were also detected in all Examples, though at lower concentrations, increasing from 0 to 24 h. The largest increase in propionate was seen in Example 4 (FucOS), reaching 10.9 mM. A considerable increase in this metabolite was observed in Example 7 (RhaOS) and Example 6 (XylOS) after 24 h fermentation. Fermentation of Example 9 (LactulOS), Example 4 (FucOS) and Example 9 (LactulOS) led to a rise in butyrate levels at 24 h, whilst enhanced levels of lactate were identified in vessels fermenting Example 5 (AraOS) and Example 6 (XylOS) after 24 h and in Example 8 (SucOS) after 8 h.

These results indicate that the GOS compositions of Examples 4-9 comprising GOS compounds with the alternative terminal monosaccharides increased *Bifidobacterium* populations. Additionally, bacteria numbers from FISH data; in combination with SCFA results from these fermentations; suggest that several of these compositions of Examples 4-9 stimulated bacteria other than the classical probiotics, *Bifidobacterium* and *Lactobacillus* and may therefore provide promising prebiotic activity.

In summary, the present invention provides a composition comprising oligosaccharide compounds, for example galactooligosaccharide compounds, which includes: (a) at least 8 wt% Gal-(β1-3)-Gal-(β1-4)-X^{a}; (b) at least 3 wt% Gal-(β1-3)-Gal-(β1-3)-X^{b}; and (c) at least 5 wt% Gal-(β1-3)-Gal-(β1-2)-X^{c}, based on the total weight of oligosaccharide compounds present in the composition; wherein X^{a}, X^{b} and X^{c} are each independently selected from monosaccharides. These compositions contain relatively high amounts of the oligosaccharide compounds (a), (b) and (c) and a relatively high amount of β1-3 Gal-Gal linkages, compared to known oligosaccharide compositions. These particular features of the composition are believed to provide benefits to the gut health of a consumer, for example due to these compositions providing an increased production of butyrate in the gut of a consumer compared to known compositions.

Although a few preferred embodiments have been shown and described, it will be appreciated by those skilled in the art that various changes and modifications might be made without departing from the scope of the invention, as defined in the appended claims.

Throughout this specification, the term "comprising" or "comprises" means including the component(s) specified but not to the exclusion of the presence of other components. The term "consisting essentially of" or "consists essentially of" means including the components specified but excluding other components except for materials present as impurities, unavoidable materials present as a result of processes used to provide the components, and components added for a purpose other than achieving the technical effect of the invention. Typically, when referring to compositions, a composition consisting essentially of a set of components will comprise less than 5% by weight, typically less than 3% by weight, more typically less than 1% by weight of non-specified components.

The term "consisting of" or "consists of" means including the components specified but excluding addition of other components.

Whenever appropriate, depending upon the context, the use of the term "comprises" or "comprising" may also be taken to encompass or include the meaning "consists essentially of" or "consisting essentially of", and may also be taken to include the meaning "consists of" or "consisting of".

For the avoidance of doubt, wherein amounts of components in a composition are described in wt%, this means the weight percentage of the specified component in relation to the whole composition referred to. For example, "wherein the oligosaccharide compounds comprise up to 35 wt% of disaccharides" means that 35 wt% of the oligosaccharide compounds in the composition is provided by disaccharides.

The optional features set out herein may be used either individually or in combination with each other where appropriate and particularly in the combinations as set out in the accompanying claims.

## Claims

1. A composition comprising oligosaccharide compounds, wherein the oligosaccharide compounds comprise:
(a) at least 8 wt% Gal-(β1-3)-Gal-(β1-4)-X^{a};
(b) at least 3 wt% Gal-(β1-3)-Gal-(β1-3)-X^{b}; and
(c) at least 5 wt% Gal-(β1-3)-Gal-(β1-2)-X^{c},
based on the total weight of oligosaccharide compounds present in the composition;
wherein X^{a}, X^{b} and X^{c} are each independently selected from monosaccharides.

2. The composition according to claim 1, wherein the oligosaccharide compounds comprise:
(d) at least 3 wt% Gal-(β1-3)-Gal-(β1-3)-Gal-(β1-4)-X^{d},
based on the total weight of oligosaccharide compounds present in the composition;
wherein X^{d}, is selected from monosaccharides;
optionally wherein the oligosaccharide compounds comprise:
(e) at least 5 wt% Gal-(β1-4)-Gal-(β1-4)-X^{e};
based on the total weight of oligosaccharide compounds present in the composition;
wherein X^{e} is selected from monosaccharides.

3. The composition according to claim 1 or claim 2, wherein:
component (a) is present in an amount up to 35 wt%;
component (b) is present in an amount up to 25 wt%; and
component (c) is present in an amount up to 25 wt%;
based on the total weight of oligosaccharide compounds present in the composition.

4. The composition according to any one of the preceding claims, wherein the ratio of the wt% of compound (a) to compound (b) is from 1:1 to 3:1;
preferably wherein the ratio of the wt% of compound (a) to compound (c) is from 1:1 to 3:1;
preferably wherein the ratio of the wt% of compound (b) to compound (c) is from 2:1 to 1:2.

5. The composition according to any one of the preceding claims, wherein X^{a}, X^{b}, X^{c}, X^{d} and X^{e} are each independently selected from glucose, fucose, arabinose, xylose, rhamnose, mannose, galactose, ribose, lyxose, allose, altrose, gulose, idose, talose, psicose, fructose, sorbose, tagatose, galactosamine, glucosamine and N-Acetylglucosamine or mixtures thereof.

6. The composition according to any one of claims 1 to 4, wherein X^{a}, X^{b}, X^{c}, X^{d} and X^{e} are each Glc.

7. The composition according to any one of claims 1 to 4, wherein X^{a}, X^{b}, X^{c}, X^{d} and X^{e} each comprise Fuc.

8. The composition according to any one of the preceding claims, wherein from 40 to 55% of the Gal-Gal linkages in the oligosaccharide compounds are 1-3 linkages.

9. The composition according to any one of the preceding claims, comprising at least 50 wt% of the oligosaccharide compounds, preferably wherein the composition is in the form of a syrup.

10. The composition according to any one of the preceding claims, comprising at least 75 wt% of the oligosaccharide compounds, preferably wherein the composition is in the form of a powder.

11. The composition according to any one of the preceding claims, wherein the oligosaccharide compounds comprise at least 25 wt% of trisaccharides;
preferably wherein the oligosaccharide compounds comprise at least 10 wt% of tetrasaccharides.

12. The composition according to any one of the preceding claims, comprising 80 wt% of trisaccharides, tetrasaccharides and higher oligosaccharides;
preferably comprising 80 wt% of trisaccharides.

13. A pharmaceutical or nutraceutical composition comprising a composition according to any one of the preceding claims and at least one carrier, excipient, or diluent.

14. Use of the composition according to any one of claim 1 to 12 as a dietary supplement;
preferably wherein the use increases butyrate production in the gut of a consumer of the dietary supplement.

15. The composition according to any one of claim 1 to 12, for use as a medicament.

## Patentansprüche

1. Zusammensetzung, umfassend Oligosaccharidverbindungen, wobei die Oligosaccharidverbindungen Folgendes umfassen:
(a) mindestens 8 Gew.-% Gal-(β1-3)-Gal-(β1-4)-X^{a};
(b) mindestens 3 Gew.-% Gal-(β1-3)-Gal-(β1-3)-X^{b}; und
(c) mindestens 5 Gew.-% Gal-(β1-3)-Gal-(β1-2)-X^{c},
jeweils bezogen auf das Gesamtgewicht der in der Zusammensetzung vorhandenen Oligosaccharidverbindungen;
wobei X^{a}, X^{b} und X^{c} jeweils unabhängig aus Monosacchariden ausgewählt sind.

2. Zusammensetzung nach Anspruch 1, wobei die Oligosaccharidverbindungen Folgendes umfassen:
(d) mindestens 3 Gew.-% Gal-(β1-3)-Gal-(β1-3)-Gal-(β1-4)-X^{d},
bezogen auf das Gesamtgewicht der in der Zusammensetzung vorhandenen Oligosaccharidverbindungen;
wobei X^{d} aus Monosacchariden ausgewählt ist;
gegebenenfalls wobei die Oligosaccharidverbindungen Folgendes umfassen:
(e) mindestens 5 Gew.-% Gal-(β1-4)-Gal-(β1-4)-X^{e};
bezogen auf das Gesamtgewicht der in der Zusammensetzung vorhandenen Oligosaccharidverbindungen;
wobei X^{e} aus Monosacchariden ausgewählt ist.

3. Zusammensetzung nach Anspruch 1 oder Anspruch 2, wobei:
Komponente (a) in einer Menge bis zu 35 Gew.-% vorhanden ist;
Komponente (b) in einer Menge bis zu 25 Gew.-% vorhanden ist; und
Komponente (c) in einer Menge bis zu 25 Gew.-% vorhanden ist,
jeweils bezogen auf das Gesamtgewicht der in der Zusammensetzung vorhandenen Oligosaccharidverbindungen.

4. Zusammensetzung nach einem der vorhergehenden Ansprüche, wobei das Verhältnis der Gew.-% von Verbindung (a) zu Verbindung (b) 1:1 bis 3:1 beträgt;
vorzugsweise wobei das Verhältnis der Gew.-% von Verbindung (a) zu Verbindung (c) 1:1 bis 3:1 beträgt;
vorzugsweise wobei das Verhältnis der Gew.- % von Verbindung (b) zu Verbindung (c) 2:1 bis 1:2 beträgt.

5. Zusammensetzung nach einem der vorhergehenden Ansprüche, wobei X^{a}, X^{b}, X^{c}, X^{d} und X^{e} jeweils unabhängig aus Glucose, Fucose, Arabinose, Xylose, Rhamnose, Mannose, Galactose, Ribose, Lyxose, Allose, Altrose, Gulose, Idose, Talose, Psicose, Fructose, Sorbose, Tagatose, Galactosamin, Glucosamin und N-Acetylglucosamin oder Mischungen davon ausgewählt sind.

6. Zusammensetzung nach einem der Ansprüche 1 bis 4, wobei es sich bei X^{a}, X^{b}, X^{c}, X^{d} und X^{e} jeweils um Glc handelt.

7. Zusammensetzung nach einem der Ansprüche 1 bis 4, wobei X^{a}, X^{b}, X^{c}, X^{d} und X^{e} jeweils Fuc umfassen.

8. Zusammensetzung nach einem der vorhergehenden Ansprüche, wobei 40 bis 55 % der Gal-Gal-Verknüpfungen in den Oligosaccharidverbindungen 1-3-Verknüpfungen sind.

9. Zusammensetzung nach einem der vorhergehenden Ansprüche, umfassend mindestens 50 Gew.-% der Oligosaccharidverbindungen, wobei die Zusammensetzung vorzugsweise in Form eines Sirups vorliegt.

10. Zusammensetzung nach einem der vorhergehenden Ansprüche, umfassend mindestens 75 Gew.-% der Oligosaccharidverbindungen, wobei die Zusammensetzung vorzugsweise in Form eines Pulvers vorliegt.

11. Zusammensetzung nach einem der vorhergehenden Ansprüche, wobei die Oligosaccharidverbindungen mindestens 25 Gew.-% Trisaccharide umfassen;
vorzugsweise wobei die Oligosaccharidverbindungen mindestens 10 Gew.-% Tetrasaccharide umfassen.

12. Zusammensetzung nach einem der vorhergehenden Ansprüche, umfassend 80 Gew.-% Trisaccharide, Tetrasaccharide und höhere Oligosaccharide;
vorzugsweise umfassend 80 Gew.-% Trisaccharide.

13. Pharmazeutische oder nutrazeutische Zusammensetzung, umfassend eine Zusammensetzung nach einem der vorhergehenden Ansprüche und mindestens ein Träger-, Hilfs- oder Verdünnungsmittel.

14. Verwendung der Zusammensetzung nach einem der Ansprüche 1 bis 12 als Nahrungsergänzungsmittel;
vorzugsweise wobei durch die Verwendung die Butyratproduktion im Darm eines Konsumenten des Nahrungsergänzungsmittels gesteigert wird.

15. Zusammensetzung nach einem der Ansprüche 1 bis 12 zur Verwendung als Arzneimittel.

## Revendications

1. Composition comprenant des composés oligosaccharides, dans laquelle les composés oligosaccharides comprennent :
(a) au moins 8 % en poids de Gal-(β1-3)-Gal-(β1-4)-X^{a} ;
(b) au moins 3 % en poids de Gal-(β1-3)-Gal-(β1-3)-X^{b} ; et
(c) au moins 5 % en poids de Gal-(β1-3)-Gal-(β1-2)-X^{c},
par rapport au poids total de composés oligosaccharides présents dans la composition ;
X^{a}, X^{b} et X^{c} étant chacun indépendamment choisis parmi les monosaccharides.

2. Composition selon la revendication 1, dans laquelle les composés oligosaccharides comprennent :
(d) au moins 3 % en poids de Gal-(β1-3)-Gal-(β1-3)-Gal-(β1-4)-X^{d},
par rapport au poids total de composés oligosaccharides présents dans la composition ;
X^{d} étant choisi parmi les monosaccharides ;
éventuellement dans laquelle les composés oligosaccharides comprennent :
(e) au moins 5 % en poids de Gal-(β1-4)-Gal-(β1-4)-X^{e} ;
par rapport au poids total de composés oligosaccharides présents dans la composition ;
X^{e} étant choisi parmi les monosaccharides.

3. Composition selon la revendication 1 ou la revendication 2, dans laquelle :
le composant (a) est présent en une quantité allant jusqu'à 35 % en poids ;
le composant (b) est présent en une quantité allant jusqu'à 25 % en poids ; et
le composant (c) est présent en une quantité allant jusqu'à 25 % en poids ;
par rapport au poids total de composés oligosaccharides présents dans la composition.

4. Composition selon l'une quelconque des revendications précédentes, dans laquelle le rapport du % en poids du composé (a) à celui du composé (b) va de 1:1 à 3:1 ;
de préférence dans laquelle le rapport du % en poids du composé (a) à celui du composé (c) va de 1:1 à 3:1 ;
de préférence dans laquelle le rapport du % en poids du composé (b) à celui du composé (c) va de 2:1 à 1:2.

5. Composition selon l'une quelconque des revendications précédentes, dans laquelle X^{a}, X^{b}, X^{c}, X^{d} et X^{e} sont chacun indépendamment choisis parmi le glucose, le fucose, l'arabinose, le xylose, le rhamnose, le mannose, le galactose, le ribose, le lyxose, l'allose, l'altrose, le gulose, l'idose, le talose, le psicose, le fructose, le sorbose, le tagatose, la galactosamine, la glucosamine, la N-acétylglucosamine ou les mélanges de ceux-ci.

6. Composition selon l'une quelconque des revendications 1 à 4, dans laquelle X^{a}, X^{b}, X^{c}, X^{d} et X^{e} sont chacun Glc.

7. Composition selon l'une quelconque des revendications 1 à 4, dans laquelle X^{a}, X^{b}, X^{c}, X^{d} et X^{e} comprennent chacun Fuc.

8. Composition selon l'une quelconque des revendications précédentes, dans laquelle de 40 à 55 % des liaisons Gal-Gal dans les composés oligosaccharides sont des liaisons 1-3.

9. Composition selon l'une quelconque des revendications précédentes, comprenant au moins 50 % en poids des composés oligosaccharides, de préférence la composition étant sous la forme d'un sirop.

10. Composition selon l'une quelconque des revendications précédentes, comprenant au moins 75 % en poids des composés oligosaccharides, de préférence la composition étant sous la forme d'une poudre.

11. Composition selon l'une quelconque des revendications précédentes, dans laquelle les composés oligosaccharides comprennent au moins 25 % en poids de trisaccharides ;
de préférence, dans laquelle les composés oligosaccharides comprennent au moins 10 % en poids de tétrasaccharides.

12. Composition selon l'une quelconque des revendications précédentes, comprenant 80 % en poids de trisaccharides, tétrasaccharides et oligosaccharides supérieurs ;
de préférence comprenant 80 % en poids de trisaccharides.

13. Composition pharmaceutique ou nutraceutique comprenant une composition selon l'une quelconque des revendications précédentes et au moins un véhicule, excipient ou diluant.

14. Utilisation de la composition selon l'une quelconque des revendications 1 à 12 en tant que complément alimentaire ;
de préférence l'utilisation augmentant la production de butyrate dans l'intestin d'un consommateur du complément alimentaire.

15. Composition selon l'une quelconque des revendications 1 à 12, destinée à être utilisée en tant que médicament.
